(19) Europäisches Patentamt
European Patent Office
Office européen des brevets

(11) **EP 3 851 516 A1**

(12) **EUROPÄISCHE PATENTANMELDUNG**

(43) Veröffentlichungstag:
**21.07.2021 Patentblatt 2021/29**

(51) Int Cl.:
***C12M 3/06*** *(2006.01)*     ***C12M 1/00*** *(2006.01)*
***C12M 1/34*** *(2006.01)*

(21) Anmeldenummer: **20152016.0**

(22) Anmeldetag: **15.01.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
**KH MA MD TN**

(71) Anmelder:
• **Fraunhofer Gesellschaft zur Förderung der angewandten Forschung e.V.**
  **80686 München (DE)**
• **Universität zu Lübeck**
  **23562 Lübeck (DE)**

(72) Erfinder:
• **Zille, Marietta**
  **23552 Lübeck (DE)**
• **Palumbo, Alex**
  **23560 Lübeck (DE)**
• **Grüning, Philipp**
  **23966 Wismar (DE)**

(74) Vertreter: **Altmann Stößel Dick Patentanwälte PartG mbB**
**Dudenstrasse 46**
**68167 Mannheim (DE)**

(54) **VERFAHREN UND VORRICHTUNG ZUR KULTIVIERUNG UND UNTERSUCHUNG VON NEURONEN**

(57)     Die vorliegende Erfindung befasst sich mit einer mikrofluidischen Testvorrichtung zur Kultivierung und/oder Untersuchung von Neuronen. Insbesondere betrifft die Erfindung eine mikrofluidische Testvorrichtung zur Kultivierung und/oder Untersuchung von Neuronen, umfassend mindestens eine Trägerplatte und einen Körper, wobei der Körper eine Mehrzahl an mikrofluidische Einheiten aufweist, wobei jede mikrofluidische Einheit mindestens zwei sich gegenüberliegende Kammern umfasst, dadurch gekennzeichnet, dass der Körper monolithisch ausgestaltet ist. Weiterhin wird ein Verfahren zur Untersuchung von Neuronen bereitgestellt, umfassend die Schritte Generieren mikroskopischer Bilddaten von Neuronen in mindestens einer mikrofluidischen Einheit, wobei die Neuronen jeweils in einer mikrofluidischen Einheit vorliegen, wobei von einer mikrofluidischen Einheit mindestens ein mikroskopisches Bild erzeugt wird, wobei jedes mikroskopische Bild eine Mehrzahl an Pixeln aufweist, Generieren von sekundären Bilddaten unter Verwendung eines Bildverarbeitungsalgorithmus, wobei der Bildverarbeitungsalgorithmus für jedes Pixel der generierten Bilder mindestens eine Klassenzugehörigkeit bestimmt und Ausgeben und/oder Erhalten automatisch generierter sekundärer Bilddaten zum Zustand der Neurone. Schließlich wird ein System zur Untersuchung von Neuronen und ein Verfahren zur Kultivierung von Neuronen bereitgestellt.

Fig. 1

EP 3 851 516 A1

**Beschreibung**

Die vorliegende Erfindung befasst sich mit einer mikrofluidischen Testvorrichtung zur Kultivierung und/oder Untersuchung von Neuronen; einem Verfahren zur Untersuchung von Neuronen und einem System zur automatischen Untersuchung von Neuronen. Insbesondere betrifft die Erfindung eine mikrofluidische Testvorrichtung zur Kultivierung und/oder Untersuchung von Neuronen, umfassend mindestens eine Trägerplatte und einen Körper, wobei der Körper eine Mehrzahl an mikrofluidische Einheiten aufweist, wobei jede mikrofluidische Einheit mindestens zwei sich gegenüberliegende Kammern umfasst, dadurch gekennzeichnet, dass der Körper monolithisch ausgestaltet ist. Weiterhin wird ein Verfahren zur Untersuchung von Neuronen bereitgestellt, umfassend die Schritte Generieren mikroskopischer Bilddaten von Neuronen in mindestens einer mikrofluidischen Einheit, wobei die Neuronen jeweils in einer mikrofluidischen Einheit vorliegen, wobei von einer mikrofluidischen Einheit mindestens ein mikroskopisches Bild erzeugt wird, wobei jedes mikroskopische Bild eine Mehrzahl an Pixeln aufweist, Generieren von sekundären Bilddaten unter Verwendung eines Bildverarbeitungsalgorithmus, wobei der Bildverarbeitungsalgorithmus für jedes Pixel der generierten Bilder mindestens eine Klassenzugehörigkeit bestimmt und Ausgeben und/oder Erhalten automatisch generierter sekundärer Bilddaten zum Zustand der Neurone. Schließlich wird ein System zur Untersuchung von Neuronen und ein Verfahren zur Kultivierung von Neuronen bereitgestellt.

Untersuchungen von Neuronen und deren kabelartigen und/oder filamentartigen Auswüchsen, auch Nervenfortsätze oder Neurite genannt, ist Gegenstand zahlreicher Forschungsvorhaben, die sich mit dem Verständnis und der therapeutischen Behandlung des menschlichen und/oder tierischen Nervensystems beschäftigen.

Nervenfortsätze sind kabelartige Auswüchse von Zellkörpern von Neuronen, die Reize zum und vom Gehirn leiten. Die Funktion von Nervenfortsätzen ergibt sich aus einer komplexen Physiologie, die eine Vielzahl Molekülen zur Bereitstellung von Energie, Stabilität und Reizweiterleitung beinhaltet. Viele dieser Moleküle müssen von den Zellkörpern zu den weiter entfernten Teilen der Nervenfortsätze transportiert werden. Unter Nervenfortsätzen können Axone und Dendrite verstanden werden, die jeweils unterschiedliche Funktionen erfüllen können. Die Morphologie von Axonen und Dendriten ist divers und komplex. Axone und/oder Dendrite können unterschiedlichen Längen, Durchmesser und Verästelungen aufweisen. Zusätzlich zur Untersuchung physiologischer Parameter der Axone und/oder Dendrite liegt ein Hauptaugenmerk der Forschung in der Untersuchung von Schädigungen der Axone (axonale Degeneration). Axonale Degeneration beim Menschen kann beispielsweise zu Bewegungseinschränkung bis hin zu Bewegungsunfähigkeit führen, die Wahrnehmung der Umgebung beeinflussen und das Denkvermögen beschränken. Die Untersuchung der Degeneration von Nervenfortsätzen insbesondere von axonaler Degeneration ist erforderlich, um neue Wirkstoffe oder andere therapeutische Verfahren zu entwickeln und zu testen. Im Rahmen industrieller Anwendungen ist die Untersuchung axonaler Degeneration bislang eine Hürde in der Entwicklung neuer Wirkstoffe und Therapien für derzeit unheilbare Erkrankungen wie Querschnittslähmung, Alzheimer und Schlaganfall, bei denen die axonale Degeneration ein wichtiger Prozess im Rahmen der Erkrankung darstellt.

Eine entscheidende Rolle dabei spielt, dass mikroskopische Untersuchungen von Prozessen in Nervenfortsätzen unabhängig von deren Zellkörpern in Zellkultur zurzeit nicht mit einem hinreichenden Durchsatz möglich sind. Es gibt bislang Systeme, die in einem Objekt verbaut sind und die separate Untersuchung von zellulären Vorgängen an Axonen ermöglichen (auch Campenot Chambers oder Axon Isolation Device genannt; siehe beispielsweise Li, et al. 2014). Diese lassen aber nur Einzeluntersuchungen und keine systematischen Untersuchen zu, da diese extrem aufwendig wären.

Ein zweites Problem ist, dass die systematische Auswertung biologischer Prozesse an Axonen eine Software erfordert, die morphologische Veränderungen zellulärer Bestandteile quantitativ analysieren kann. Dies bedeutet, dass nicht nur spezifisch Nervenfortsätze, insbesondere Axone, erkannt werden müssen, sondern auch das Ausmaß der axonalen Veränderungen erkannt werden muss.

Morphologische Veränderungen über die Zeit können mithilfe von Zeitverlaufsexperimenten und/oder Zeitraffermikroskopie aufgenommen werden, welche große Mengen an Daten produzieren, sodass eine manuelle Analyse kaum durchführbar, aber auch fehleranfällig wäre, da die Bewertung subjektiv erfolgen würde und nur mit entsprechender Expertise machbar wäre. Fluoreszenzfärbungen verbessern zwar den Kontrast zwischen Zielstrukturen und Hintergrund, können aber lediglich zu einem Zeitpunkt durchgeführt werden, wobei eine zeitliche Auflösung nur in unterschiedlichen Devices möglich ist, oder mittels genetisch veränderter Nervenfortsätze, die beispielsweise mit einem Fluoreszenzmolekül markiert sind. Jedoch sind diese genetischen Veränderungen in Nervenzellen nur limitiert möglich, arbeits- und kostenintensiv und können auch die Funktionen die Nervenfortsätze beeinträchtigen. Des Weiteren können die axonale Physiologie und Degeneration mittels dieser experimentellen Anwendungen nicht umfassend untersucht werden, da der oberservierende zeitliche Ablauf an denselben Nervenfortsätzen ausschlaggebend für das Verständnis dieser Prozesse ist.

Deswegen bedarf es eines automatischen Verfahrens, das spezifisch Nervenfortsätze in mikroskopischen Aufnahmen, wie beispielsweise in der Phasen Kontrast-Mikroskopie, erkennt und quantifizieren kann, vorzugsweise ohne dass es einer Manipulation, beispielsweise genetischen Manipulation, des biologischen Materials bedarf.

Es wäre daher wünschenswert, eine mikrofluidische Testvorrichtung und ein Verfahren zur Kultivierung und/oder Untersuchung von Neuronen bereitzustellen, welche die Nachteile und/oder Limitationen bekannter Testvorrichtungen und Verfahren zumindest weitgehend vermeiden. Insbesondere soll eine schnelle und einfache Untersuchung ermöglicht werden, die automatisch und objektiv ablaufen kann und somit einen hohen Durchsatz ermöglicht.

Diese Aufgabe wird adressiert durch eine mikrofluidische Testvorrichtung, ein Verfahren zur Kultivierung und/oder Untersuchung von Neuronen und ein System zur automatischen Untersuchung von Neuronen mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nichtausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

Die vorliegende Erfindung schlägt eine mikrofluidische Testvorrichtung zur Kultivierung und/oder Untersuchung von Neuronen, vorzugsweise zur Untersuchung von Axonen, vor. Die mikrofluidische Testvorrichtung umfasst mindestens eine Trägerplatte und einen Körper, wobei der Körper eine Mehrzahl an mikrofluidischen Einheiten aufweist. Jede mikrofluidische Einheit umfasst mindestens zwei sich gegenüberliegenden Kammer. Der Körper der Testvorrichtung ist monolithisch ausgestaltet. Der Begriff monolithisch kann in diesem Zusammenhang so verstanden werden, dass der Körper aus einem Stück geformt worden ist und/oder aus einem Stück besteht.

Eine Mehrzahl an mikrofluidischen Einheiten kann vorzugsweise eine Anzahl von 2 bis 200 mikrofluidische Einheiten bedeuten, insbesondere eine Anzahl von 6 bis 100. Vorzugsweise kann der Körper mindestens 6, mehr bevorzugt mindestens 12, noch mehr bevorzugt mindestens 16 mikrofluidische Einheiten aufweisen. Vorzugsweise kann die mikrofluidische Testvorrichtung aus einer Trägerplatte und einem Körper aufweisend eine Mehrzahl an mikrofluidischen Einheiten, beispielsweise mindestens 16 mikrofluidische Einheiten, bestehen. Mehr bevorzugt kann die Mehrzahl an mikrofluidischen Einheiten zusammen unter Verwendung einer einzigen Gussform geformt werden, um den monolithischen Körper zu bilden. Noch mehr bevorzugt kann die Mehrzahl an mikrofluidischen Einheiten zusammen unter Verwendung einer Gussform, die Mulden entsprechend der Mehrzahl oder Anzahl der mikrofluidischen Einheiten aufweist, geformt werden. Beispielsweise kann die mikrofluidische Testvorrichtung einen Körper aus einem geeigneten Material umfassen, in dem die mikrofluidischen Einheiten ausgebildet sind.

Geeignete Materialien für den Körper der mikrofluidischen Testvorrichtung können sein: Glas, wie beispielsweise Quarzglas, Bleiglas, Kalknatronglas, Borsilikaglas, Silicon, Quarz, Keramik; ein Polymer auf Siliziumbasis, beispielsweise Polydimethylsiloxan (PDMS); Elastomere, beispielsweise Thermoset Polyester und thermoplastische Polymere wie Polystyrol, Polycarbonat, Polyoxymethylen, Polyethylenterephthalat (PET), Polyetherketon (PEEK), Polypropylen (PP), Polymethacrylat, Polymethylmethacrylat (PMMA), Polyethyleneglycol-Diacrylat, Teflon, Perfluoroalkoxy-Polymer, Tetrafluo-rethylen-Hexafluorpropylen-Copolymer, Polyfluoropolyetherdiol-Methacrylat, Polyurethan; zusammengesetzte Materialeien wie zyklische Olefin-Polymere, eine Mischung aus PMMA/PDMS, eine Mischung aus Papier/PDMS; Papier; und/oder Hydrogele. PDMS kann ein bevorzugtes Material sein.

Wie oben erwähnt, kann der Körper der Testvorrichtung besonders bevorzugt durch Verwendung einer Gussform, die Mulden für die mikrofluidischen Einheiten aufweist, gebildet werden. Die Ausbildung der mikrofluidischen Einheiten in einem gemeinsamen monolithischen Körper kann vorteilhaft sein, da die einzelnen mikrofluidischen Einheiten fest zueinander lokalisiert sind und so ein automatisiertes Aufnehmen von mikroskopischen Bilder mittels Hochdurchsatz- und/oder Screening-Mikroskopen ermöglicht werden kann. Beispielsweise können die mikrofluidischen Einheiten parallel untersucht werden. Insbesondere können Zeitverlaufsuntersuchungen möglich und/oder vereinfacht werden, die das parallele und/oder sequentielle Aufnehmen mikroskopischer Bilder erfordern. Weiterhin kann durch das Vorhandensein einer Mehrzahl an mikrofluidischen Einheiten auf einer Testvorrichtung die Verwendung von Mehrkanalpipetten ermöglicht werden, was zusätzlich eine Zeitersparnis bedeuten kann und somit Ressourcen schonen kann. Somit kann der experimentelle Durchsatz im Vergleich zu herkömmlichen mikrofluidischen Testvorrichtungen, die keine Parallelisierung der mikrofluidischen Einheiten ermöglichen, erhöht werden.

Vorzugsweise kann die Testvorrichtung mindestens 2, mindestens 6, mindestens 8, mindestens 16, mindestens 24, mindestens 36, oder mindestens 48 mikrofluidische Einheiten oder weiter bevorzugt bis zu 96 oder mehr mikrofluidische Einheiten aufweisen.

Verfahren zur Herstellung der mikrofluidischen Testvorrichtung sind dem Fachmann bekannt und untenstehend beschrieben.

Vorzugweise kann der Körper auf der Trägerplatte fixiert sein. Die Fixierung (Bonding) kann mittels Plasmabehandlung erfolgen. Dabei können im PDMS Silanolgruppen (Si-OH) geformt werden, die bei Kontakt mit Hydroxid (OH), Karbonsäure (COOH) und/oder Ketonen der Trägerplatte (z. B. Glas) unter Abspaltung von einem Wassermolekül eine irreversible Bindung als Si-O-Si eingehen können (Xiong et al. (2014).

Eine mikrofluidische Einheit umfasst mindestens zwei sich gegenüberliegende Kammern. Die sich gegenüberliegenden Kammer einer mikrofluidischen Einheit können jeweils ein Kompartiment aufweisen, das sich in Richtung der anderen Kammer erstreckt. Die zwei sich gegenüberliegenden Kammern können so ausgestaltet sein, dass sich in einer ersten der beiden Kammern die Zellkörper der Neuronen befinden und sich in der dieser Kammer gegenüberliegenden Kammer, der zweiten Kammer, die Nervenfortsätze der Neuronen, vorzugsweise die Axone, befinden. Diese können mit Signalempfängerzellen, wie beispielsweise Gliazellen, Muskelzellen, Endothelzellen, Drüsenzellen, Darmzellen, und/oder Tumorzellen in Kontakt stehen. Vorzugsweise kann das Kompartiment, das sich von der ersten Kammer in Richtung der zweiten Kammer erstreckt als erstes Kompartiment bezeichnet werden und weiter bevorzugt kann ein weiteres Kompartiment, das sich von der zweiten Kammer in Richtung der ersten Kammer erstreckt als zweites oder axonales Kompartiment bezeichnet werden.

Vorzugsweise besteht eine mikrofluidische Einheit aus zwei sich gegenüberliegenden Kammern, die fluidtechnisch in Verbindung stehen. Dabei weisen die Kompartimente bevorzugt Mikrokanäle auf, mehr bevorzugt stellen die Mikrokanäle eine Verbindung zwischen den beiden Kompartimenten und/oder Kammern dar, beispielsweise eine fluidtechnische Verbindung, die vorzugsweise das Fließen der für die Zellkultur notwendigen Flüssigkeiten zwischen den Kompartimenten ermöglichen kann.

Die Testvorrichtung gemäß der vorliegenden Erfindung weist vorzugsweise einen Boden auf, der die Kompartimente und/oder die Kammern auf der Unterseite verschließt, mehr bevorzugt verschließt der Boden, die Kammern, die Kompartimente und die Mikrokanäle auf der Unterseite.

Vorzugsweise wird der Boden der Testvorrichtung von der Trägerplatte gebildet. Weiter bevorzugt wird der Boden von einer Trägerplatte umfassend oder bestehend aus mindestens einem Material ausgewählt aus Glas und/oder Kunststoff gebildet. Besonders bevorzugt wird der Boden von der Trägerplatte von einem Material ausgewählt aus: Silicon; Glas, wie beispielsweise Quarzglas, Bleiglas, Kalknatronglas, Borsilikaglas; Quarz; Keramik; Elastomere, wie beispielsweise PDMS, Thermoset Polyester, thermoplastische Polymere wie Polystyrol, Polycarbonat, Polyoxymethylen, Polyethylen, Polymethylmethacrylat, Polyethylenglycol-Diacrylat, Polyethylenterephthalat, mit Glycol modifiziertes Polytetrafluorethylen, Polyurethan, gebildet.

Die Trägerplatte kann insbesondere eine Beschichtung aufweisen; beispielsweise eine Beschichtung umfassend eine Substanz ausgewählt aus einem Protein, einem Peptid, einem Kohlenhydrat, einer Aminosäure. Die Beschichtung kann die Haftung der Neuronen oder anderer Zellen, wie beispielsweise der oben genannten Signalempfängerzellen, auf der Trägerplatte erhöhen und/oder deren Wachstum verbessern. Geeignete Beschichtungen sind dem Fachmann bekannt und können beispielsweise Laminin, Lysin, Ornithin, Gelatine, Kollagen, Fibronektin, Elastin, Extrazellulärmatrix-Coating, Wachstumsfaktoren und/oder andere umfassen.

Vorzugsweise kann die Trägerplatte eine Breite von 80 bis 100 mm und/oder eine Länge von 110 bis 140 mm und/oder eine Höhe von 0,5 bis 2 mm haben; besonders bevorzugt kann eine Breite von 86 mm, eine Länge von 128 mm und eine Höhe von 1 mm sein. Die genannten Ausmaße sind vorteilhaft, da sie das Einspannen in einen herkömmlichen Mikroskopiertisch ermöglichen, insbesondere in einen Mikroskopiertisch eines Hochdurchsatz Mikroskops, wie beispielsweise eines BioTek Lionheart, BioTek Cytation, Perkin Elmer Operetta CLS, oder Perkin Elmer Opera Phenix CLS.

Der Begriff "mikrofluidische Testvorrichtung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff bezeichnet jede Vorrichtung die einen mikrofluidischen Fluss zwischen fluidtechnisch miteinander verbundenen Elementen zur Aufnahme von Flüssigkeiten, ermöglicht; insbesondere zwischen fluidtechnisch miteinander verbundenen Kammern im Sinne der vorliegenden Erfindung. Unter "Kammer" kann im Sinne der vorliegenden Erfindung jede Art von Hohlräume oder andere Einheiten geeignet für die Aufnahme von Flüssigkeiten verstanden werden; beispielsweise können Kammern auch Wells von Mikrotiterplatten umfassen. "Kammern" im Sinne der vorliegenden Erfindung sind untenstehend weiter ausgeführt.

Die Testvorrichtung gemäß der vorliegenden Erfindung kann weiterhin mindestens eines der folgenden Materialien umfassen oder daraus bestehen: Glas, wie beispielsweise Quarzglas, Bleiglas, Kalknatronglas, Borsilikaglas, Silicon, Quarz, Keramik; ein Polymer auf Siliziumbasis, beispielsweise Polydimethylsiloxan (PDMS); Elastomere, beispielsweise Thermoset Polyester und thermoplastische Polymere wie Polystyrol, Polycarbonat, Polyoxymethylen, Polynmethylmethacrylat (PMMA), Polyethyleneglycol-Diacrylat, Polyethylenterephthalat, Teflon, Perfluoroalkoxy-Polymer, Tetrafluorethylen-Hexafluorpropylen-Copolymer, Polyfluoropolyetherdiol-Methacrylat, Polyurethan; zusammengesetzte Materialien wie zyklische Olefin-Polymere, eine Mischung aus PMMA/PDMS, eine Mischung aus Papier/PDMS; Papier; und/oder Hydrogele.

Die Begriffe "Nervenfortsatz" und "Neurit" haben im Zusammenhang mit der vorliegenden Anmeldung dieselbe Bedeutung und werden daher austauschbar verwendet. Bei Neuriten handelt es sich um Fortsätze von Neuronen, beispielsweise um Axone und/oder Dendrite. Axone können insbesondere für die Weiterleitung elektrischer Reize an andere Neuronen und/oder Effektorzellen und/oder Signalempfängerzellen in Zielgeweben verantwortlich sein. Demgegenüber können Dendrite für die Aufnahme von elektrischen Reizen, die z.B. über Axone weitergeleitet werden, verantwortlich sein.

Der Begriff "fluidtechnische Verbindung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf Verbindungen zwischen zwei Kammern einer Testvorrichtung beziehen, die einen Flüssigkeitsdurchfluss erlauben. Insbesondere können solche Verbindungen durch Mikrokanäle verwirklicht werden.

Der Begriff "Mikrokanäle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff "Mikrokanäle" kann, ohne Beschränkung, sich insbesondere auf Kanäle zwischen zwei Kammern beziehen, die vorzugsweise eine maximale Höhe von 5 μm, weiterhin bevorzugt eine maximale Breite von 10 μm und außerdem bevorzugt eine maximale Länge von 4350 μm oder mehr bevorzugt eine maximale Länge von 1500 μm aufweisen. Insbesondere können darunter solche Kanäle verstanden werden, die eine Höhe von 4 bis 6 μm, eine Breite von 8 bis 12 μm und eine Länge von 750 bis 1500 μm aufweisen. Besonders bevorzugt können Mikrokanäle mit einer Höhe von 5 μm, einer Länge von maximal 1000 μm und einer Breite von 10 μm sein.

Der Begriff "mikrofluidische Einheit", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff "mikrofluidische Einheit" kann, ohne Beschränkung, sich insbesondere auf eine Einheit der Testvorrichtung beziehen, wie beispielsweise eine mikrofluidische Einheit, die mindestens zwei z.B. sich gegenüberliegende Kammern umfasst, die fluidtechnisch verbunden sind. Die fluidtechnische Verbindung kann vorzugsweise aus Mikrokanälen bestehen. Vorzugsweise besteht zwischen den sich gegenüberliegenden Kammern ein mikrofluidischer Fluss. Mehr bevorzugt kann zwischen den Kammern ein Gefälle bestehen, beispielsweise der Flüssigkeiten, in derart, dass sich der Flüssigkeitsstand zwischen den Kammern unterscheidet, das heißt eine der Kammern einen höheren Flüssigkeitsstand aufweist als die andere, so dass ein Flüssigkeitsstrom von der Kammer mit dem höheren Flüssigkeitsstand hinüber zu der Kammer mit dem niedrigeren Flüssigkeitsstand bestehen kann. Der mikrofluidische Fluss kann insbesondere durch das Zugeben von unterschiedlichen Mengen an Flüssigkeit zu den sich gegenüberliegenden Kammern erreicht und/oder eingestellt werden. Beispielsweise kann in eine der gegenüberliegenden Kammer 10, 20, 30, 40 oder auch 50 Vol.-% mehr Flüssigkeit zugegeben werden wie in die andere der gegenüberliegenden Kammern. Dadurch kann beispielsweise ein mikrofluidischer Fluss in Richtung der anderen Kammer erzeugt werden.

Unter dem Begriff "mikrofluidisch" kann im Sinne der vorliegenden Anmeldung verstanden werden, dass die flüssigkeitsleitenden Komponenten der Vorrichtung (z.B. die Mikrokanäle) bevorzugt einen Maßstab im Mikrometerbereich aufweisen. In den flüssigkeitsleitenden Komponenten der Vorrichtung kann die Kontrolle der Flüssigkeiten vorzugsweise basierend auf Kapillarkräften, die aufgrund der Ausmaße der flüssigkeitsleitenden Komponenten der Vorrichtung, wie beispielsweise der Mikrokanäle, im Mikrometerbereich wirken, stattfinden.

Der Begriff "mikrofluidischer Fluss" wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff "mikrofluidischer Fluss" kann insbesondere eine durch Kapillarkräfte bewirkte Fließrichtung einer Flüssigkeit in der mikrofluidischen Testvorrichtung bezeichnen. Insbesondere kann der mikrofluidische Fluss eine Fließrichtung zwischen den mindestens zwei sich gegenüberliegenden Kammer bewirken; ganz besonders kann der mikrofluidische Fluss eine Fließrichtung, beispielsweise einer Nährflüssigkeit oder eines Nährmediums, von einer ersten Kammer der mikrofluidischen Einheit, in der sich mindestens ein Zellkörper eines Neurons befinden kann, in Richtung einer zweiten Kammer der mikrofluidischen Einheit, in der sich mindestens ein von dem Zellkörper ausgehender Nervenfortsatz, insbesondere ein Axon befinden kann, bewirken.

Unter "Kapillarkraft" kann das von Flüssigkeiten gezeigte Verhalten, das sie bei Kontakt mit Kapillaren, z. B. engen Röhren, Mikrokanälen, Spalten oder Hohlräumen in Feststoffen zeigen, verstanden werden. Diese Effekte können durch die Oberflächenspannung von Flüssigkeiten selbst und die Grenzflächenspannung zwischen Flüssigkeiten und der festen Oberfläche hervorgerufen werden. Insbesondere kann unter dem Begriff "Kapillarkraft" im Sinne der vorliegenden Erfindung eine Adhäsionskraft verstanden werden, die zwischen einer Flüssigkeit und der Wandung einer sie umgebenden Röhre und/oder eines sie umgebenen Mikrokanals wirken kann. Diese Kraft kann insbesondere die Steighöhe und den Transport der Flüssigkeit innerhalb der Röhre und/oder des Mikrokanals und/oder innerhalb der mikrofluidischen Testvorrichtung beeinflussen.

Der Begriff "Kompartiment", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff "Kompartiment" kann, ohne Beschränkung, sich insbesondere auf eine Aussparung beziehen, die sich von der jeweiligen Kammer zu der anderen gegenüberliegenden Kammer hin erstreckt. Ein Kompartiment im Sinne der vorliegenden Erfindung kann beispielsweise eine Breite von 2 bis 15 mm aufweisen; vorzugsweise eine Breite von 4 bis 8 mm; besonders bevorzugt eine Breite von 4 bis 5 mm aufweisen.

Ein Kompartiment im Sinne der vorliegenden Erfindung kann nach oben und unten und auf zwei Seiten geschlossen sein; auf der Seite, die zu der gegenüberliegenden Kammer hin orientiert ist, kann das Kompartiment mindestens eine Öffnung aufweisen; diese Öffnung kann an Mikrokanäle anschließen; auf der Seite, die zu der jeweiligen Kammer hin orientiert ist, kann eine direkte Verbindung zu dieser Kammer bestehen. Vorzugsweise können die Kompartimente mit der jeweiligen Kammer direkt verbunden sein. Unter einer "direkten Verbindung" kann in diesem Zusammenhang eine Öffnung, die sich über die gesamte Breite des Kompartiments erstreckt, verstanden werden. Somit können die Ausmaße der Öffnung durch die Ausmaße des Kompartiments begrenzt sein. Beispielhafte Ausmaße der Öffnung können sein: eine maximale Höhe von 14,9 mm, eine maximale Breite von 15 mm, und/oder eine maximale Länge von 2,5 mm. Bevorzugt kann die Öffnung eine Höhe von 100 bis 200 $\mu$m, eine Breite von 0,5 bis 8 mm, weiter bevorzugt eine Breite von 4 bis 5 mm, und/oder eine Länge von 1 bis 2,3 mm aufweisen.

Die Kompartimente können weiterhin Mikrokanäle aufweisen. Insbesondere, können die Mikrokanäle eine Verbindung zwischen den beiden Kompartimenten oder den Kammern darstellen. Vorzugsweise können die Mikrokanäle ausgehend von einem Kompartiment einer Kammer zu einem Kompartiment der gegenüberliegenden Kammer eine Verbindung ausbilden. Noch mehr bevorzugt kann durch die Mikrokanäle eine fluidtechnische Verbindung zwischen den sich gegenüberliegenden Kompartimenten ausgebildet werden. Eine fluidtechnische Verbindung kann vorteilhaft sein, da sie das Bereitstellen eines mikrofluidischen Flusses zwischen den Kammern ermöglichen kann und somit eine Richtung für das Wachstum der Nervenfortsätze vorgegeben werden kann. Weiterhin können die Mikrokanäle aufgrund ihrer geringen Ausmaße vorteilhaft sein, da sie den Durchtritt nur einzelner oder einiger weniger Nervenfortsätze ermöglichen können. Durch eine solche Separation kann die Untersuchung einzelner oder nur weniger Nervenfortsätze bewerkstelligt werden.

Vorzugsweise können die Mikrokanäle voneinander einen Abstand von maximal 100 $\mu$m aufweisen. Mehr bevorzugt können die Mikrokanäle einen Abstand von maximal 75 $\mu$m, mehr bevorzugt von maximal 50 $\mu$m auf weisen. Noch mehr bevorzugt kann der Abstand zwischen einzelnen Mikrokanälen zwischen 20 und 50 $\mu$m betragen. Dieser Abstand kann vorteilhaft sein, da so die Nervenfortsätze in verschiedenen Mikrokanälen separat im Mikroskop abgebildet werden können. Vorzugsweise können sich die Mikrokanäle auf derselben Ebene befinden, mehr bevorzugt auf derselben Fokusebene. Noch mehr bevorzugt können die Mikrokanäle nach unten hin direkt an die Trägerplatte anschließen.

Vorzugsweise sind die Kompartimente über eine Vielzahl von Mikrokanälen miteinander verbunden. Bevorzugt beträgt die Anzahl der Mikrokanäle zwischen den Kompartimenten maximal 250; besonders bevorzugt 20 bis 200, noch mehr bevorzugt 50 bis 100. Eine solche Anzahl kann den Vorteil haben, dass ein ausreichender mikrofluidischer Fluss bewerkstelligt werden kann und Nervenfortsätze, insbesondere Axone, entlang des mikrofluidischen Flusses von einem oder mehreren Zellkörpern der Neurone in der ersten Kammer ausgehend in die Mikrokanäle einwachsen können und/oder sich vorzugsweise bis in die gegenüberliegende zweite Kammer erstrecken können. Eine geringere Anzahl an Mikrokanälen kann den Nachteil haben, dass der mikrofluidische Fluss nicht ausreichend sein kann, um ein gerichtetes Wachstum der Axone zu bewerkstelligen. Ein weiterer Vorteil kann darin bestehen, dass die beschriebene Anzahl an Mikrokanälen zu einem Abstand der Mikrokanäle untereinander führen kann, der für die mikroskopische Analyse gut geeignet sein kann; beispielsweise dadurch, dass die Axone, die durch einen Mikrokanal gewachsen sind, separat betrachtet werden können. Eine höhere Anzahl an Mikrokanälen kann den Nachteil haben, dass es zu einer zu hohen Anzahl und somit zu einer hohen Dichte der Axone in der zweiten Kammer kommen kann und so eine separate mikroskopische Analyse der Axone aufgrund einer zu hohen Dichte der Axone nicht mehr möglich sein kann.

Die Kompartimente und/oder die Mikrokanäle können weiterhin eine Überdeckung aufweisen. Die Überdeckung kann derart gestaltet sein, dass sie die Neuronen und/oder Axone größtenteils von oben abdeckt. Das Vorhandensein einer Überdeckung kann vorteilhaft sein, da beim Auswechseln des Kulturmediums, beispielsweise mit einer Mehrkanalpipette, die Zellkörper der Neurone und/oder die Axone größtenteils von der Überdeckung geschützt werden und daher weniger leicht verletzt werden können.

Die Kammern einer mikrofluidischen Einheit haben vorzugsweise eine zylindrische Form. Noch mehr bevorzugt weisen die Kammern eine zylindrische Form auf, vorzugsweise können die Kammern einen Durchmesser von 1 bis 10 mm, mehr bevorzugt von 2 bis 8 mmm aufweisen. Ein solcher Durchmesser kann vorteilhaft sein, um ein Medienvolumen zu erreichen, dass die Kultivierung von Zellkörpern und Nervenfortsätzen ermöglichen kann. Weiterhin können die Kammern eine maximale Höhe von 15 mm aufweisen, vorzugsweise können die Kammern eine Höhe von 1 bis 15 mmm aufweisen. Eine solche Höhe kann vorteilhaft sein, um ein Volumen zu erreichen, beispielsweise ein Volumen von mindestens 20 $\mu$l oder mindestens 50 $\mu$l, das die Kultivierung von Zellkörpern und Nervenfortsätzen ermöglichen kann.

Typischerweise ist eine Kammer gemäß der vorliegenden Erfindung ausgestaltet, um ein Volumen von 20 bis 350 $\mu$l, insbesondere ein Volumen von 50 bis 200 $\mu$l, zu umfassen. Solche Volumina können insbesondere vorteilhaft sein, da sie für das Einstellen des mikrofluidischen Flusses geeignet sein können. Für eine Kultivierung von Zellen und oder Zellkulturen im Sinne der vorliegenden Erfindung kann ein Volumen von 50 bis 350 $\mu$l notwendig sein. Genannte Volumen können vorteilhaft sein, da die Kultivierung der Zellen/Zellkulturen und/oder die Versorgung der Zellen/Zellkulturen mit Nährstoffen über einen Zeitraum von 1 bis 60 Tagen ermöglicht werden kann. Während der Kultivierung können aus angesäten Zellkörpern der Neurone Axone auswachsen, die sich durch die Mikrokanäle hindurch von der einen Kammer, vorzugsweise von der ersten Kammer, in die gegenüberliegende Kammer, vorzugsweise in die zweite Kammer, hin erstrecken können. Geeignete Medien, Kulturmedien, Nährmedien oder Nährflüssigkeiten sind dem Fachmann bekannt und beispielsweise in Muramatsu and Yamashita (2013) veröffentlicht.

Die Begriffe "Kulturmedien", "Nährmedien" oder "Nährflüssigkeiten" beziehen sich auf Medien, die für die Anzucht und/oder Kultivierung von Zellen, insbesondere von Neuronen, geeignet sind und werden hier austauschbar verwendet. Für Nervenzellen besonders geeignete Nährmedien können insbesondere die folgenden Medien ein schließen: Minimum Essential Medium (MEM) + Glutamax enthaltend 10% Pferdeserum und/oder Dulbecco's Modified Eagle's Medium (DMEM) enthaltend 10% Pferdeserum, Wachstumsfaktoren und/oder DMEM-F12 Medium enthaltend 10% Pferdeserum, 1mM Natriumpyruvat und/oder Eagle's MEM enthaltend 0,5 mM Glutamin, 20 mM Glucose und/oder Neurobasal A Medium enthaltend 2% B-27 oder B-27 Plus Supplement, 1mM Natriumpyruvat und/oder Neurobasal Plus Medium enthaltend 2% B-27 oder B-27 Plus Supplement, 1mM Natriumpyruvat (kommerziell erhältlich beispielsweise bei Thermo Fisher Scientific) und/oder Neuronal Base Medium enthaltend 2% N21-MAX Media Supplement, Wachstumsfaktoren (kommerziell erhältlich beispielsweise bei R&D Systems).

Der Abstand der gegenüberliegenden Kammern kann vorzugsweise zwischen 2 und 10 mm betragen. Entsprechend der Abstände zwischen den Kammern kann sich die Länge der die Kammern verbindenden Mikrokanäle verlängern. Daraus kann entsprechend eine größere Distanz entstehen, die ein Axon beim Hineinwachsen von der einen Kammer in die gegenüberliegende Kammer überbrücken muss. Die Kultivierungszeiten der Axone können sich so entsprechend verlängern. Zu große Abständen zwischen den gegenüberliegenden Kammern können nachteilig sein, dass sich die Kultivierungszeiten der Axone zu stark verlängern können.

Die vorliegende Erfindung stellt weiterhin ein Verfahren zur Kultivierung von Neuronen bereit. Das Verfahren umfasst die folgenden Schritte:

a) Bereitstellen einer mikrofluidischen Testvorrichtung gemäß der vorliegenden Erfindung;
b) Aussäen von Neuronen in mindestens eine der Kammern und/oder Kompartimente, so dass mindestens eine angesäte Kammer entsteht,
c) Inkontaktbringen zumindest eines Teils der angesäten Kammern mit einem Kulturmedium, und
d) Kultivieren der Neuronen.

Der Begriff "Kultivierung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff "Kultivierung" kann, ohne Beschränkung, sich insbesondere auf die Anzucht von Zellen oder Zellkulturen in einem geeigneten Wachstumsmedium unter Bedingungen, die das Wachstum der jeweiligen Zellen oder Zellkulturen erlauben, beziehen. Als Kultivierung kann insbesondere die Anzucht der Zellen oder Zellkulturen bei einer Luftfeuchtigkeit von vorzugsweise 95%, bei einem $CO_2$-Gehalt von vorzugsweise 5 Vol.-% und/oder bei einer Temperatur von mindestens 20 °C bis maximal 45 °C verstanden werden, besonders geeignet ist eine Temperatur von 37°C. Durch die Kultivierung unter den genannten Bedingungen kann vorteilhafterweise insbesondere Verdunstung vermieden und der pH-Wert konstant gehalten werden. Die Anzucht kann beispielsweise in einem geeigneten kommerziell erhältlichen Inkubator erfolgen, wie beispielsweise $CO_2$-Inkubatoren mit Heißluftsterilisation und hitzesterilisierbarem $CO_2$-Sensor der CB Serie von Binder (Tuttlingen, Deutschland). Als geeignetes Medium kann eines oder können mehrere der oben genannten Kulturmedien verwendet werden.

Schritt a) des Bereitstellens einer mikrofluidischen Testvorrichtung kann insbesondere das Erstellen einer mikrofluidischen Testvorrichtung mittels geeigneter lithographischer Mittel und/oder Verfahren umfassen. Diese Verfahren und Mittel sind dem Fachmann bekannt. Insbesondere kann es sich dabei um photo- und/oder softlithographische Mittel und/oder Verfahren handeln, wie aus Whitesides et al., Annu Rev Biomed Eng. 2001; 3:335-373 bekannt. Um die mikrofluidische Testvorrichtung zu erstellen kann eine geeignete Gussform, beispielsweise eine Gussform aus Epoxid, verwendet werden. Vorzugsweise wird in diese Gussform eine Mischung der Monomeren eines geeigneten Materials, vorzugsweise PDMS eingebracht und mit geeigneten Mitteln polymerisiert. Diese Mittel sind dem Fachmann beispielsweise aus Whitesides et al. (2001) bekannt.

Das Aussäen der Neuronen in Schritt b) kann insbesondere mit einer Zelldichte von 1000 bis 20000 Zellen pro mm$^2$ bezogen auf die Grundfläche des Kompartiments, vorzugsweise bei einer Zelldichte von 6000 Zellen pro mm$^2$ bezogen auf die Grundfläche des Kompartiments erfolgen. Des Weiteren kann das Aussäen zusätzliche Schritte, wie beispielsweise das Anhaften der Zellen an die Testvorrichtung, beinhalten. Das Aussäen kann weiterhin die vorausgehende Isolierung von Nervenvorläuferzellen, wie beispielsweise die Isolierung von primären Neuronen aus dem Gehirn, Rückenmark oder Spinalganglien eines geeigneten Spenderorganismuses, wie beispielsweise Mensch, Maus, Ratte, Kaninchen, und/oder weiteren, umfassen. Auch eine vorausgehende Gewinnung induzierter pluripotenter Stammzellen (iPSC) aus beispielweise menschlichen Fibroblasten, die dann erst zu Neuronen ausdifferenziert werden können, kann denkbar sein.

Schritt c) des Inkontaktbringens zumindest eines Teils der angesäten Kammern mit einem Kulturmedium kann vorzugsweise das Einbringen eines geeigneten Kulturmediums mittels einer herkömmlichen Laborpipette oder einer Mehrkanalpipette umfassen. Vorzugsweise kann Schritt c) das Einbringen von mindestens 20

μl eines geeigneten Kulturmediums in jede der gegenüberliegenden Kammern und/oder Kompartimente umfassen. Bevorzugt kann eine unterschiedliche Menge an Kulturmedium in die gegenüberliegenden Kammern der mikrofluidischen Einheiten eingebracht werden. Mehr bevorzugt kann die Menge an Kulturmedium, das in die erste Kammer eingebracht wird, die Menge des Kulturmediums, das in die zweite Kammer eingebracht wird, übersteigen. Noch mehr bevorzugt kann die Menge an Kulturmedium, das in die in die erste Kammer eingebracht wird, die Menge des Kulturmediums das in die zweite Kammer eingebracht wird um 10 Vol.-% bis 100 Vol.-%, beispielsweise um 50 Vol.-%, übersteigen in Bezug auf die Menge an Kulturmedium in der zweiten Kammer. Eine geeignete Menge an Kulturmedium kann beispielsweise zwischen 20 μl und 350 μl betragen. Durch das Einbringen unterschiedlicher Mengen an Kulturmedium in die gegenüberliegenden Kammern kann der mikrofluidische Fluss erzeugt werden. Dies kann vorteilhaft sei, da so ein gerichtetes Wachstum der Axone ausgehend vom Zellkörper in der ersten Kammer hin zur zweiten Kammer erfolgen kann.

Das in die beiden gegenüberliegenden Kammern eingebrachte Kulturmedium kann dasselbe sein, alternativ können aber auch unterschiedliche Kulturmedien in die erste und die zweite Kammer eingebracht werden. Ein solches Vorgehen kann vorteilhaft sein, um spezifische Auswirkungen auf einen Teil, das Axon oder den Zellkörper des Neurons untersuchen zu können.

Schritt d) umfasst das Kultivieren der Neuronen. Dieser Schritt kann insbesondere die Inkubation der Neuronen bei einer geeigneten Temperatur, geeigneter Luftfeuchte und/oder einem geeigneten $CO_2$-Gehalt umfassen. Insbesondere kann darunter das Inkubieren bei 37°C in einer 5 Vol.-% $CO_2$ Atmosphäre bei 95% Luftfeuchtigkeit verstanden werden.

Vorzugsweise kann das Verfahren zur Kultivierung von Neuronen weiterhin umfassen: e) Co-Kultivieren von weiteren Zellen, beispielsweise Gliazellen, Muskelzellen, Endothelzellen, Drüsenzellen, Darmzellen, Tumorzellen. Das Co-Kultivieren weiterer Zellen kann die Untersuchung von Zell-Zell-Interaktionen ermöglichen und kann daher vorteilhaft sein.

Das Verfahren zur Kultivierung kann weiterhin umfassen: Schritt f) wiederholen zumindest der Schritte c) und d) und/oder weitere Schritte umfassen.

Weiterhin stellt die vorliegende Erfindung ein Verfahren zur Untersuchung von Neuronen bereit. Das Verfahren umfasst die folgenden Schritte:

a) Generieren mikroskopischer Bilddaten von Neuronen in mindestens einer mikrofluidischen Einheit, wobei die Neuronen jeweils in einer mikrofluidischen Einheit vorliegen; wobei von einer mikrofluidischen Einheit mindestens ein mikroskopisches Bild erzeugt wird, wobei jedes mikroskopische Bild eine Mehrzahl an Pixeln aufweist;
b) Generieren von sekundären Bilddaten unter Verwendung eines Bildverarbeitungsalgorithmus, wobei der Bildverarbeitungsalgorithmus für jedes jeden Pixel der in Schritt a) generierten Bilder mindestens eine Klassenzugehörigkeit bestimmt; und

c) Ausgeben und/oder Erhalten automatisch generierter sekundärer Bilddaten zum Zustand der Neurone.

Das Verfahren kann weiterhin die folgenden optionalen Schritte umfassen:

cc) Erzeugen von Daten zum Zustand der Neurone aus den sekundärer Bilddaten; und/oder
d) Zusammenfügen eines digital aufbereiteten Bildes basierend auf den sekundären Bilddaten;
e) Darstellen der automatisch generierten sekundären Bilddaten des digital aufbereiteten Bildes auf einer Bildwiedergabevorrichtung; und/oder
f) Ausgeben und/oder Erhalten automatisch generierter tertiärer und/oder quartärer Bilddaten.

In Schritt a) können die Neuronen vorzugsweise jeweils in einer der mikrofluidischen Einheiten der Testvorrichtung gemäß der vorliegenden Erfindung vorliegen. Vorzugsweise können von einer mikrofluidischen Einheit mehrere mikroskopische Bilddaten erzeugt werden. Weiterhin bevorzugt können die Bilddaten mindestens die beiden gegenüberliegenden Kammern und/oder die Kompartimente abbilden. Besonders bevorzugt können die Bilddaten die gesamte mikrofluidische Einheit, beispielsweise die gesamte mikrofluidische Einheit in ihrer flächenmäßigen Ausdehnung, abbilden. Beispielsweise können bei einer bevorzugten mikroskopischen Auflösung von 10-fach von den gegenüberliegenden Kammern, den Kompartimenten und/oder der mikrofluidischen Einheit 1 bis 12 Bilder erzeugt werden, vorzugsweise 3 bis 8 Bilder. Die bevorzugte Anzahl der Bilder und/oder Bilddaten und/oder die mikroskopische Auflösung kann je nach Zweck und/oder Fragestellung der Untersuchung variieren. Eine möglichst geringe Anzahl an Bildern kann vorteilhaft sein, da so Zeit und Ressourcen, wie zum Beispiel Speicherkapazitäten, gespart werden können.

Der Begriff "gesamte mikrofluidische Einheit" soll so verstanden werden, dass insbesondere mindestens die beiden sich gegenüberliegenden Kammer und die Mikrokanäle umfasst sind, besonders bevorzugt können die beiden sich gegenüberliegenden Kammer, die Kompartimente und die Mikrokanäle umfasst sein.

Weiterhin kann Schritt a) ein Generieren von Bilddaten über einen Zeitverlauf umfassen. Das Generieren von Bilddaten über einen Zeitverlauf kann beispielsweise das Erzeugen von Bilddaten jeweils nach Ablauf eines bestimmten Zeitintervalls umfassen, beispielsweise jeweils nach 30 oder nach 60 Minuten über einen Zeitraum von mehreren Stunden und/oder Tagen. Je nach Zweck und/oder Fragestellung der jeweiligen Untersuchung können auch kürzere oder längere Zeitintervalle und/oder Zeiträume verwendet werden.

Vorzugsweise kann das Generieren der mikroskopischen Bilddaten in Schritt a) das Aufnehmen von mikroskopischen Bilddaten umfassen, bevorzugt Daten von lichtmikroskopischen Aufnahmen und/oder fluoreszenzmikroskopischen Aufnahmen; mehr bevorzugt Daten von Phasenkontrastaufnahmen.

Für das Generieren der mikroskopischen Bilddaten besonders geeignet können insbesondere Lichtmikroskope, wie beispielsweise Lichtmikroskope ausgebildet für die Phasenkontrastmikroskopie, und/oder Fluoreszenzmikroskope sein. Zum Generieren der Bilddaten können auch Hochdurchsatzmikroskope und/oder Screening-Mikroskope verwendet werden. Besonders bevorzugt kann die Verwendung eines Hochdurchsatzmikroskops ausgebildet für die Phasenkontrastmikroskopie sein, wie beispielsweise BioTek Lionheart, BioTek Cytation, Perkin Elmer Operetta CLS, oder Perkin Elmer Opera Phenix CLS. Die Verwendung von Hochdurchsatz- oder Screening-Mikroskopen kann vorteilhaft sein, da eine weitgehend automatisierte Erzeugung der Bilddaten möglich werden kann.

In Schritt a) wird weiterhin von einer mikrofluidischen Einheit mindestens ein mikroskopisches Bild erzeugt, wobei jedes mikroskopische Bild eine Mehrzahl an Pixeln aufweist. Eine Mehrzahl an Pixeln kann in diesem Zusammenhang 64x64 bis 1024x1024 Pixel bedeuten, vorzugsweise 512x512. Wenn die Anzahl an Pixeln zu klein ist, kann die Klassifizierung durch den Bildverarbeitungsalgorithmus negativ beeinflusst werden. Vorzugsweise kann das Erzeugen des mikroskopischen Bildes die Speicherung des Bildes in einer Auswerteeinheit umfassen.

Bevorzugt kann das Generieren der Bilddaten in Schritt a) und b) mikroskopische Bilddaten von Axonen umfassen.

Der Begriff "automatisch generierte sekundäre Bilddaten" bezieht sich in diesem Zusammenhang insbesondere auf die unter Verwendung des Bildverarbeitungsalgorithmus generierten sekundären Bilddaten.

Das Generieren von sekundären Bilddaten unter Verwendung eines Bildverarbeitungsalgorithmus in Schritt b) kann vorzugsweise einen Bildverarbeitungsalgorithmus umfassen, welcher anhand von mikroskopischen Bilddaten mit bekannten Informationen trainiert wurde. Mehr bevorzugt kann das Generieren von sekundären Bilddaten unter Verwendung eines Bildverarbeitungsalgorithmus in Schritt b) einen Machine-Learning-Algorithmus umfassen, welcher anhand von mikroskopischen Bilddaten mit bekannten Informationen trainiert wurde. Weiterhin bevorzugt kann der Bildverarbeitungsalgorithmus jedem Pixel die Wahrscheinlichkeit einer Klassenzugehörigkeit zuordnen. Noch mehr bevorzugt kann das Generieren von sekundären Bilddaten mit einem Bildverarbeitungsalgorithmus in Schritt b) einen Deep Learning Algorithmus, welcher anhand von mikroskopischen Bilddaten mit bekannten Informationen trainiert wurde, umfassen; am weiterhin bevorzugt kann das Generieren von sekundären Bilddaten mit einem Bildverarbeitungsalgorithmus in Schritt b) ein Convolutional Neural Network (CNN) umfassen, welches anhand von mikroskopischen Bilddaten mit bekannten Informationen trainiert wurde. Am meisten bevorzugt kann der Bildverarbeitungsalgorithmus in Schritt b) ein CNN sein. Insbesondere kann das Generieren von sekundären Bilddaten in schritt b) automatisch ablaufen.

Der Begriff "mikroskopische Bilddaten mit bekannten Informationen", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf Bilddaten beziehen, die mittels eines Mikroskops aufgenommen wurden und Pixel umfassen.

Weiterhin können aus den Bilddaten mikroskopische Bilder bestehend aus einer Mehrzahl an Pixeln erzeugt werden. Unter einer Mehrzahl an Pixeln können hier beispielsweise 64x64, 128x128, 256x256, 512x512 oder 1024x1024 Pixel pro Bild verstanden werden.

Beispielsweise kann der Begriff "mikroskopische Bilddaten mit bekannten Informationen" sich auf Bilddaten oder Bilder umfassend Pixel beziehen, wobei zu jedem einzelnen Pixel mindestens Informationen über eine Klassenzugehörigkeit bekannt sind. Vorzugsweise ist mindestens die Zugehörigkeit ausgewählt aus einer von zwei Klassen, mehr bevorzugt ausgewählt aus den Klassen Hintergrund und Neuron, noch mehr bevorzugt ausgewählt aus den Klassen Hintergrund und Axon. Weiterhin kann die Klassenzugehörigkeit ausgewählt aus mehr als zwei Klassen, vorzugsweise aus zwei bis zehn Klassen einschließlich drei, vier, fünf, sechs, sieben, acht, neun oder zehn Klassen, ausgewählt sein. Es können aber auch mehr als zehn Klassen möglich sein. Besonders bevorzugt kann die Klassenzugehörigkeit aus vier Klassen ausgewählt sein. Insbesondere bevorzugt kann die Zugehörigkeit aus einer der folgenden Klassen ausgewählt sein: Hintergrund, Axon, axonales Fragment, axonaler Spheroid. Mikroskopische Bilddaten mit bekannten Informationen können beispielsweise über manuelle Markierung von Bildelementen mittels geeigneter Software erhalten werden. Zu hierfür geeigneter Software zählen unter anderem pixelbasierte Grafikprogramme wie GIMP (GNU Image Manipulation Program; https://docs.gimp.org/2.10/de/). Zur Erstellung mikroskopischer Bilddaten mit bekannten Informationen können beispielsweise Bildelemente umfassend die gewünschten Zellen oder deren Bestandteile, wie beispielsweise Neuronen oder Axone, in einem existierenden mikroskopischen Bild von Hand markiert werden. Vorzugsweise können die Bildelemente aus zusammenhängenden Pixeln bestehen oder diese umfassen. Weiterhin vorzugsweise umfassen die existierenden mikroskopischen Bilder mikroskopische Aufnahmen von Neuronen und/oder Axonen vorzugsweise Phasenkontrast-Aufnahmen oder fluoreszenzmikroskopische Aufnahmen.

Die markierten Bildelemente können dann vorzugsweise der Klasse Neuron oder Axon zugeordnet sein; mehr bevorzugt können die markierten Bildelemente einer von drei Klassen: Axon, axonaler Spheroid oder axonales Fragment zugeordnet sein. Die von Hand (manuell) markierten Bildelemente können insbesondere die Grundwahrheit für die Klassenzuordnung der Pixel darstellen.

Unmarkierte Bildelemente können vorzugsweise der Klasse Hintergrund zugeordnet sein. Vorzugsweise kann die Markierung auf Basis eines Schwellwerts gesetzt werden. Geeignete Schwellwertverfahren sind dem Fachmann bekannt (Meijering, 2012). Der Schwellwert kann insbesondere einen Helligkeitswert, und/oder Werte wie die Klassenwahrscheinlichkeit umfassen. Dieser Wert kann bevorzugt durch den Bildverarbeitungsalgorithmus festgelegt werden. Mehr bevorzugt kann die Markierung auf Basis der größten Wahrscheinlichkeit, beispielsweise auf Basis eines Schwellwerts von 0,5 bei den Klassen Axon/Hintergrund, zugeordnet werden.

Beispielsweise können sich bei 4 Klassen für jedes Pixel vier Werte ergeben, die sich alle zu 1 aufsummieren können; z.B. (0.8, 0.05, 0.05, 0.1) für ein Pixel. Vorzugsweise reflektiert der höchste Wert, die höchste Wahrscheinlichkeit zur Zugehörigkeit zu einer bestimmten Klasse; mehr bevorzugt bestimmt der höchste Wahrscheinlichkeitswert die Klassenzughörigkeit.

Die genannten Schwellwert-Verfahren können vorzugsweise auf den primären/und oder sekundären Bilddaten angewendet werden. Im Falle von nur 2 Klassen, beispielsweise Neuron (oder Axon) und Hintergrund, kann vorzugsweise ein einziger Schwellwert ausreichend sein. Dann können sich nur zwei Werte Pro Pixel (x, y) ergeben, wobei gilt: x+y=1 und somit y = 1 - x. Man kann vorzugsweise als Ausgabe ein Bild der Größe [Eingangshöhe x Eingangsbreite x 1] bekommen, wobei jedes Pixel einen Wert zwischen 0 und 1 annehmen kann. Es kann beispielsweise einen Schwellwert t (beispielsweise 0.5) bestimmt werden und die Aussage "alle Pixel <= t gehören zu Klasse 0 alle anderen zu Klasse 1" kann getroffen werden.

Zusätzlich können die bekannten Informationen der Bilddaten weitere Informationen umfassen insbesondere zu: axonalem Wachstum, axonaler Degeneration, axonaler Remodelierung, und/oder axonaler Regeneration.

Das Trainieren des Bildverarbeitungsalgorithmus anhand von mikroskopischen Bilddaten mit bekannten Informationen kann beispielsweise in einer U-Net basierten Architektur stattfinden. Insbesondere kann eine U-Net Variante mit einem VGG16 Encoder oder ResNet, DenseNet, Hourlass, MobileNet, oder EfficientNet verwendet werden. Vorzugsweise können das Trainieren eine Veränderung der mikroskopischen Bilddaten umfassen; dafür kann beispielsweise eine Standardisierung der mikroskopischen Bilddaten stattfinden (preprocessing) und/oder eine Spiegelung der mikroskopischen Bilddaten (data augmentation; O. Ronneberger, P.

Fischer, and T. Brox, "U-net: Convolutional networks for biomedical image segmentation," in International Conference on Medical image computing and computer-assisted intervention. Springer, 2015, pp. 234-241). Das Trainieren kann mehr bevorzugt die Verwendung von einem oder von mehreren Modelle umfassen.

Besonders bevorzugt umfasst das Trainieren mehrere Wiederholungen mit mindestens 5, noch mehr bevorzugt mit mindestens 10 Bilddatensätzen. Dabei kann ein Bilddatensatz vorzugsweise eine mikroskopische Aufnahme umfassend die mikroskopischen Bilddaten mit bekannten Informationen, wie obenstehend ausgeführt, umfassen oder daraus bestehen. Dieser Bilddatensatz kann als Trainingsprobe bezeichnet werden. Eine geeignete Anzahl an Bilddatensätzen oder Trainingsproben kann 10 bis 100 Bilddatensätze umfassen, bevorzugt mindestens 15, mehr bevorzugt mindestens 30; eine Anzahl von 10 Bilddatensätzen kann bereits ausreichend sein. Ein Trainieren mit der genannten Anzahl an Bilddatensätzen oder Trainingsproben kann vorteilhaft sein, da es zu einer ausreichend genauen Zuordnung von Pixeln aus unbekannten mikroskopischen Bildern oder mikroskopischen Bilddaten führen kann. Die Verwendung einer größeren Anzahl kann den Nachteil haben, dass das Trainieren mehr Zeit und mehr Aufwand erfordert.

Vorzugsweise kann jede Trainingsprobe mindestens je ein Bild umfassen, welches aus Pixeln besteht oder diese umfasst und wobei zu jedem einzelnen Pixel mindestens Informationen über eine Klassenzugehörigkeit bekannt sind. Der Bildverarbeitungsalgorithmus kann weiterhin vorzugsweise für mindestens 5 und maximal 1000 Epochen unter Verwendung eines geeigneten Optimierers, wie beispielsweise eines Adam, SGD, AdaDelta, Lamb, oder RMSProb Optimierers; einer Batchgröße von 2 bis 32; und einer Lernrate zwischen 10 und 10$^{-6}$, insbesondere mittels Backpropagation, trainiert worden sein. Die Bilddaten mit bekannten Informationen können beispielsweise mittels ImageNet Mittelwert und Standardabweichung normalisiert werden. Weiterhin kann vorzugsweise eine Inferenz ausgeführt worden sein. Die oben genannten Verfahren sind dem Fachmann beispielsweise aus Ronneberger et al. bekannt.

Zusätzlich kann beim Trainieren mit Bilddaten aus Zeitverlaufsexperimenten ein Medianfilter auf der zeitlichen Ebene mit einem geeigneten Zeitfenster durchgeführt werden, beispielsweise 3,5 h bei 7 Bildern.

Weiterhin kann für das Trainieren der Datensatz mittels data augmentation künstlich vergrößert werden. Eine solche Vergrößerung des Datensatzes kann insbesondere zufälliges Ausscheiden der Bilddaten umfassen, beispielsweise mit einer Größe von 448x448 Pixeln und/oder zufälliges Drehen und Umklappen der Bilddatensätze entlang der vertikalen oder horizontalen Achse oder beiden umfassen. Vorzugsweise kann das Drehen der Bilddatensätze zwischen 0 und 270 Grad erfolgen, mehr bevorzugt mit 0°, 90°, 180° und/oder 270°. Weiterhin können beispielsweise Verfahren wie additive noise, Farbinvertierung, Farbkanäle vertauschen, affine Transformationen, Image Warping, Löschen einzelner Pixel oder Bereiche und/oder zufällige Helligkeits- und/oder Kontrast-Transformation zur Augmentation des Datensatzes verwendet werden.

Das Trainieren kann insbesondere ein Verfahren zur stochastischen Optimierung umfassen, insbesondere ein stochastisches Gradientenverfahren, wie stochastisch gradient descent (SGD).

In Schritt b) des Verfahrens zur Untersuchung von Neuronen werden sekundäre Bilddaten unter Verwendung eines Bildverarbeitungsalgorithmus generiert.

Der Bildverarbeitungsalgorithmus bestimmt in Schritt b) für jedes Pixel der in Schritt a) generierten Bilddaten mindestens eine Klassenzugehörigkeit. Die in Schritt a) generierten Bilddaten können als primäre Bilddaten bezeichnet werden. Mehr bevorzugt umfassen diese primären Bilddaten Pixel oder bestehen daraus. Beispielsweise können die in Schritt a) generierten Bilddaten Bilder, die Pixel umfassen oder daraus bestehen. Die Bilder können beispielsweise aus 512x512 Pixeln oder aus 1024x1024 Pixeln bestehen.

Vorzugsweise kann der Bildverarbeitungsalgorithmus die Zugehörigkeit ausgewählt aus einer von zwei Klassen bestimmen, mehr bevorzugt die Zugehörigkeit ausgewählt aus Hintergrund und Neuron, noch mehr bevorzugt die Zugehörigkeit ausgewählt aus Hintergrund und Axon bestimmen. Weiterhin kann der Bildverarbeitungsalgorithmus die Klassenzugehörigkeit ausgewählt aus mehr als zwei Klassen bestimmen, vorzugsweise aus zwei bis zehn Klassen einschließlich drei, vier, fünf, sechs, sieben, acht, neun oder zehn Klassen. Es können aber auch mehr als zehn Klassen möglich sein. Besonders bevorzugt kann der Bildverarbeitungsalgorithmus in Schritt b) für jedes Pixel der in Schritt a) generierten Bilddaten die Klassenzugehörigkeit zu mindestens oder zu genau vier Klassen bestimmen. Insbesondere bevorzugt kann der Bildverarbeitungsalgorithmus die Zugehörigkeit zu einer der folgenden Klassen bestimmen: Hintergrund, Axon, axonaler Spheroid, axonales Fragment. Ganz besonders bevorzugt, kann der Bildverarbeitungsalgorithmus in Schritt b) ein Verfahren der semantischen Segmentierung umfassen und/oder ausführen.

Insbesondere kann die Bestimmung zu einer Klassenzugehörigkeit für jedes Pixel der in Schritt a) generierten Bilddaten durch den Bildverarbeitungsalgorithmus die Ausgabe einer Wahrscheinlichkeit für die Zugehörigkeit zu jeder der Klassen umfassen. Vorzugsweise kann der Bildverarbeitungsalgorithmus zu jedem Pixel eine Wahrscheinlichkeit für jede Klasse ausgeben, anhand derer das Pixel einer Klasse zugeordnet werden kann. Besonders bevorzugt kann das Pixel anhand einer durch den Bildverarbeitungsalgorithmus ausgegebenen Wahrscheinlichkeit zu einer der Klassen Hintergrund oder Axon zugeordnet werden; noch mehr bevorzugt zu einer der Klassen Hintergrund, Axon, axonaler Spheroid oder axonales Fragment. Alternativ kann insbesondere eine Zuordnung zu einer der Klassen Hintergrund oder Neuron erfolgen. Weiterhin bevorzugt kann die Klasse mit dem höchsten Wert der Wahrscheinlichkeit für die Zuordnung ausschlaggebend sein; insbesondere kann der Bildverarbeitungsalgorithmus das Pixel der Klasse, für die der Wert der ausgegebenen Wahrscheinlichkeit am höchsten ist, zuordnen.

Das Generieren von sekundären Bilddaten in Schritt b) kann vorzugsweise unter Verwendung eines Convolutional Neural Network (CNN), auch als "Faltungsnetzwerk" bekannt, stattfinden. Insbesondere kann das Generieren von sekundären Bilddaten in Schritt b) unter Verwendung eines U-Net stattfinden; beispielsweise kann ein U-Net mit VGG16-Encoder, oder ResNet, DenseNet, MobileNet, EfficientNet verwendet werden. Beispielsweise können auch andere Segmentierungsarchitekturen wie Hourglass oder DeepLab verwendet werden.

Vorzugsweise kann das Generieren von sekundären Bilddaten in Schritt b) das Segmentieren der Bilddaten umfassen. Unter "Segmentieren der Bilddaten" wird im Hinblick auf die vorliegende Erfindung das Zuordnen der Bilddaten, insbesondere der Pixel, zu mindestens einer Klasse verstanden. Das Zuordnen kann, wie obenstehend beschrieben, die Bestimmung einer Klassenzugehörigkeit für jedes Pixel umfassen. Diese Bilddaten, umfassend Pixel, die einer Klassen zugeordnet sind, zu dieser gehören oder die eine Klassenzugehörigkeit haben, können im Sinne der vorliegenden Erfindung als sekundäre Bilddaten bezeichnet werden.

Unter "sekundären Bilddaten" werden insbesondere Bilddaten verstanden, die mittels des Bildverarbeitungsalgorithmus aus den oben erläuterten primären Bilddaten abgeleitet werden können. Als "Sekundäre Bilddaten" im Sinne der vorliegenden Erfindung können insbesondere Bilddaten umfassend Pixel mit mindestens einer Klassenzugehörigkeit verstanden werden. Vorzugsweise umfassen die sekundären Bilddaten mikroskopische Bilder umfassend oder bestehend aus Pixeln, wobei für jedes dieser Pixel mindestens eine Klassenzugehörigkeit bestimmt worden ist, oder bestehen daraus.

Beispielsweise kann der Bildverarbeitungsalgorithmus nach der Bestimmung der Klassenzugehörigkeit in Schritt b) in Schritt c) automatisch generierte sekundäre Bilddaten ausgeben, die ein Bild mit einer der Anzahl der Klassen entsprechenden Anzahl an Farbkanälen umfassen oder daraus bestehen. Vorzugsweise kann dabei je nach Klassenzugehörigkeit jedem Pixel ein bestimmter Farbkanal zugeordnet werden.

Das Generieren von sekundären Bilddaten kann insbesondere automatisch und/oder standardisiert ablaufen, das heißt ohne menschliches Zutun von außen; beispielsweise rein maschinell basierend auf der Wirkung des Bildverarbeitungsalgorithmus.

Das Segmentieren der Bilddaten kann insbesondere das Erstellen einer Maske anhand der sekundären Bilddaten umfassen. Die sekundären Bilddaten können vorzugsweise in Form einer Maske graphisch dargestellt werden. Unter einer "Maske" kann im Sinne der vorliegenden Erfindung ein Bildelement in einem Bild verstanden werden, dass eine Gruppe aneinander angrenzender Pixel einer Klasse umfasst oder aus dieser besteht. Dabei kann den Pixeln einer Klasse beispielsweise ein bestimmter Farbwert zugeordnet sein. So können aus den Pixeln einer Klasse zusammenhängende Bildelemente mit einem bestimmten Farbwert abgeleitet werden, die insbesondere als Maske über das ursprüngliche Bild gelegt werden können.

Besonders bevorzugt kann das Generieren von sekundären Bilddaten in Schritt b) semantisches Segmentieren der Bilddaten umfassen. Semantisches Segmentieren kann vorteilhaft sein, da es eine Klassifizierung in mehrere Klassen vornehmen kann und/oder sich unregelmäßig geformte Objekte klar erkennen lassen. Vorzugsweise kann das semantische Segmentieren bildweise angewendet werden.

Optional kann dem Trainieren in Schritt b) ein Schritt bb) des Validierens folgen. Das Validieren des Bildverarbeitungsalgorithmus kann die Verwendung eines leave-one-out cross Validierungsverfahrens oder eines k-fold cross Validierungsverfahrens umfassen.

Als Qualitätsmaß beim Validieren kann insbesondere ein Klassen-weiser F1-Score verwendet werden. Für jede Klasse kann dafür die binäre Klassifikation: "Gehört-zu-Klasse? ja/nein" betrachtet werden. Dann kann mit einer Grundwahrheit für jedes Pixel verglichen werden, wobei die Grundwahrheit den von Hand markierten Bildelementen entspricht: wahre Positive (True positives, TPs) können alle Pixel sein, die der Algorithmus der Klasse zuordnet, die auch wirklich zu der Klasse gehören. Gehören sie in Wirklichkeit nicht dazu, können es falsch Positive (False Positives, FPs) sein. Für den Fall, dass der Algorithmus aussagen sollte: "Das Pixel gehört nicht zur Klasse", in Wirklichkeit gehört dieses Pixel aber zu dieser Klasse, kann dieses Pixel ein falsch Negativ (False Negative, FN) sein. Der F1 Score kann dann anhand folgender Formel berechnet werden:

$$2 * \text{TPs} / (2 * \text{TPs} + \text{FP} + \text{FN})$$

Weiterhin kann das Validieren das Ermitteln eines Präzisionswertes (precision) und/oder eines Recall-Wertes umfassen. Der Präzisionswert (precision) kann das Verhältnis aus richtig positiven Zuordnungen (TP) geteilt durch die Summe aus richtig positiven und falsch positiven Zuordnungen (FP) bezeichnen; d.h. einer hohen Precision liegt eine niedrige falsch-positiv Rate zugrunde. Dabei kann der Präzisionswert anhand folgender Formel bestimmt werden: precision = TP/TP+FP.

Recall kann das Verhältnis aus richtig positiven Zuordnungen geteilt durch die Summe aus richtig positiven (TP) und falsch negativen Zuordnungen (FN) bezeichnen und der Sensitivität der Klassenzuordnung entsprechen. Dabei kann recall anhand folgender Formel bestimmt werden: recall = TP/TP+FN.

Für die Validierung können insbesondere die Werte für precision, recall und/oder der F1-Score mit einer Grundwahrheit für jede Klasse separat verglichen werden. Alternativ kann ein unabhängiger Datensatz, also ein Datensatz der nicht im Training verwendet wurde, für die Validierung verwendet werden. Für den Fall das mehrere Modelle trainiert wurden, kann das Modell, welches am besten auf dem Validierungsdatensatz abgeschnitten hat, verwendet werden.

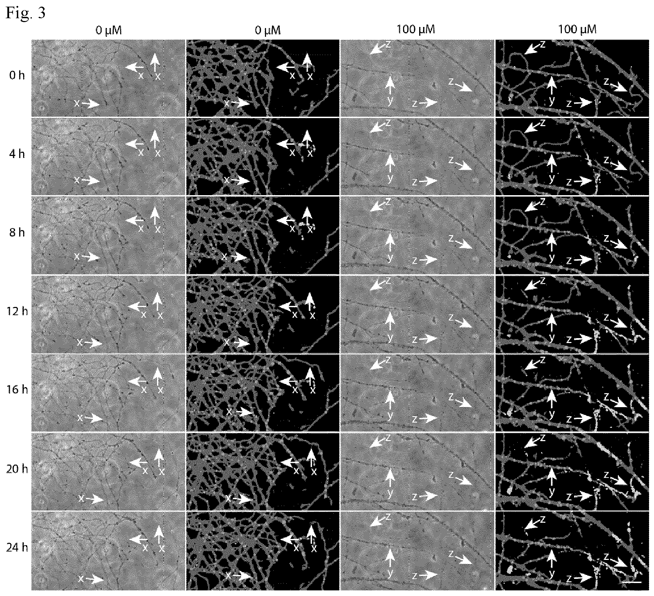

Schritt c) des Verfahrens zur Untersuchung von Neuronen umfasst das Ausgeben und/oder Erhalten automatisch generierter sekundärer Bilddaten zum Zustand der Neurone. Dabei können die durch den Bildverarbeitungsalgorithmus automatisch generierten sekundären Daten bevorzugt in Form einer graphischen Darstellung und/oder in Form einer Tabelle ausgegeben werden. Die sekundären Bilddaten können insbesondere als digital aufbereitetes Bild, wie beispielsweise als Fehlfarbenbild ausgegeben werden und/oder dargestellt werden. Vorzugsweise kann in dem digital aufbereiteten Bild je nach Klassenzugehörigkeit des Pixels dem Pixel ein bestimmter Farbwert, insbesondere ein bestimmter Grauwert zugeordnet werden und daraus beispielsweise in einem optionalen Schritt d) ein digital aufbereitetes Bild zusammengefügt werden (siehe beispielsweise Figur 3). Beispielsweise kann der Grauwert wie folgt ermittelt werden: ein Grauwertbild kann insbesondere Pixelwerte zwischen 0 und 255 aufweisen. Bei vier Klassen kann dann folgende Gleichung aufgestellt werden: 255/(4-1) = 85 (gerundet). Also können beispielsweise die Pixelwerte 0, 85, 170 und 255 verwendet werden. Der Einfachheit halber könnten auch 0, 1, 2, 3 als Pixelwerte verwendet werden.

Weiterhin können die sekundären Bilddaten beispielsweise als Tabellen ausgegeben werden. Solche Tabellen können insbesondere die Anzahl der Pixel umfassen, die einer bestimmten Klasse angehören. Weiterhin ist eine Ausgabe der sekundären Bilddaten in Form einer Grafik und/oder jeder anderen dem Fachmann bekannten Darstellungsweise denkbar.

In Schritt cc) können optional Daten zum Zustand der Neurone aus den sekundären Bilddaten erzeugt werden. Diese Daten können beispielsweise insbesondere Tabellen und/oder graphische Darstellungen zum Zustand der Neurone sein wie vorliegend erläutert.

Bevorzugt umfasst das Verfahren zur Untersuchung von Neuronen Schritt d) des Zusammenfügens eines digital aufbereiteten Bildes basierend auf den sekundären Bilddaten, wie obenstehend beschrieben. Das Zusammenfügen des digital aufbereiteten Bildes kann vorzugsweise unter Verwendung des Bildverarbeitungsalgorithmus automatisch durchgeführt werden.

Weiterhin optional kann das Verfahren zur Untersuchung von Neuronen einen Schritt e) des Darstellens der automatisch generierten sekundären Bilddaten oder des digital aufbereiteten Bildes auf einer Bildwiedergabevorrichtung umfassen. Das Darstellen des digital aufbereiteten Bildes kann vorzugsweise unter Verwendung des Bildverarbeitungsalgorithmus automatisch durchgeführt werden. Geeignete Bildwiedergabevorrichtungen sind dem Fachmann bekannt und können insbesondere Computerbildschirme, Tabletcomputer und/oder Smartphones umfassen.

Weiterhin optional kann das Verfahren zur Untersuchung von Neuronen einen Schritt f) des Ausgebens und/oder Erhaltens automatisch generierter tertiärer und/oder quartärer Bilddaten umfassen.

Der Begriff "Zustand der Neurone", wie er hier verwendet wird, ist ein weiter Begriff, dem seine gewöhnliche und gängige Bedeutung beigemessen werden soll, wie der Fachmann sie versteht. Weiterhin kann unter dem Begriff "Zustand der Neurone" insbesondere ein Zustand der Axone verstanden werden. Der Begriff ist nicht beschränkt auf eine spezielle oder angepasste Bedeutung. Der Begriff kann, ohne Beschränkung, sich insbesondere auf einen Zustand physischer Art, wie beispielsweise das Vorhandensein, die Größe, die Fläche, die Form und/oder einen Zustand biologischer Art beziehen. Unter einem Zustand biologischer Art kann insbesondere einer der folgenden Zustände verstanden werden, ausgewählt aus der Gruppe bestehend aus: Axon, insbesondere gesundes Axon; und degeneriertes Axon, insbesondere axonaler Spheroid und/oder axonales Fragment. Weiterhin kann unter dem Begriff "Zustand der Neurone" insbesondere ein Zustand der Axone verstanden werden, beispielsweise: axonales Wachstum, axonale Degeneration, axonale Remodelierung und/oder axonale Regeneration.

Im Verfahren zur Untersuchung von Neuronen gemäß der vorliegenden Erfindung kann insbesondere das Zusammenfügen des digital aufbereiteten Bildes in Schritt d) basierend auf tertiären Bilddaten abgeleitet aus den sekundären Bilddaten stattfinden. Besonders bevorzugt kann der Bildverarbeitungsalgorithmus aus den sekundären Bilddaten tertiäre Bilddaten ableiten. Unter tertiären Bilddaten kann vorzugsweise die Verteilung der Klassenzugehörigkeit der Pixel, insbesondere der Pixel eines Bildes, über einen Zeitverlauf verstanden werden. Beispielsweise kann sich die Klassenzugehörigkeit eines Pixels im Zeitverlauf jeweils zu einer anderen Klasse, vorzugsweise zu einer der anderen drei Klassen, ändern. Diese tertiären Bilddaten können optional in einen Schritt f) des Verfahrens ausgegeben oder erhalten werden.

Insbesondere kann der Bildverarbeitungsalgorithmus in Schritt d) tertiäre Bilddaten aus Zeitverlaufsexperimenten ableiten, bei denen für jeden Pixel sekundäre Bilddaten zu mehr als einem Zeitpunkt vorliegen. Dies kann insbesondere bedeuten, dass in tertiären Bilddaten für jedes Pixel die Informationen zur Klassenzugehörigkeit zu mehr als einem Zeitpunkt vorliegen. Bevorzugt können tertiäre Bilddaten die Klassenzugehörigkeit eines Pixels zu mindestens zwei verschiedenen Zeitpunkten umfassen oder daraus bestehen, mehr bevorzugt zu mindesten drei, mindestens zehn oder mindestens 100 verschiedenen Zeitpunkten. Die Informationen zu verschiedenen Zeitpunkten können insbesondere mittels Zeitverlaufsexperimenten und/oder Zeitrafferaufnahmen erfasst werden. Die Durchführung von Zeitverlaufsexperimenten und/oder Zeitrafferaufnahmen ist dem Fachmann bekannt. Zeitverlaufsexperimente können insbesondere das Generieren von mikroskopischen Bilddaten, beispielsweise von jeweils denselben Neuronen und/oder Axonen, die vorzugsweise in der mikrofluidischen Testvorrichtung vorliegen, zu bestimmten Zeitpunkten während eines Zeitverlaufs umfassen. Aus den mikroskopischen Bilddaten zu mehreren bestimmten Zeitpunkten können insbesondere Zeitrafferaufnahmen abgeleitet werden.

Das Generieren von tertiären Bilddaten in Schritt d) kann vorzugsweise unter Verwendung des gleichen neuronalen Netzwerks wie in Schritt b) oder eines anderen neuronalen Netzwerks stattfinden. Insbesondere kann Schritt d) unter Verwendung eines Recurrent Neural Network (RNN), auch als rekurrentes oder rückgekoppeltes neuronales Netz bekannt, stattfinden. Insbesondere kann in Schritt d) ein Encoder-Decoder RNN verwendet werden wie aus Bahdanau et al. bekannt.

Das Verfahren zur Untersuchung von Neuronen gemäß der vorliegenden Erfindung kann vorzugsweise das Wiederholen der Schritte a) bis c) zu bestimmten Zeitpunkten umfassen. Somit können weiterhin bevorzugt die Bilddaten der Schritte a) bis b) zu mehreren bestimmten Zeitpunkten generiert werden. Weiterhin bevorzugt kann Schritt c) des Verfahrens zur Untersuchung von Neuronen zusätzlich das Ausgeben und/oder Erhalten automatisch generierter Bilddaten zum Zustand der Neurone zu bestimmten Zeitpunkten umfassen. Bestimmte Zeitpunkte können beispielsweise den Zeitpunkt 0, als vor Beginn eines Zeitverlaufsexperiments, den Zeitpunkt nach Ablauf von 12 Stunden, und/oder den Zeitpunkt nach Ablauf von 24 Stunden umfassen. Weitere Zeitpunkte können je nach Zweck oder Fragestellung der Untersuchung denkbar sein.

Vorzugsweise können aus den tertiären Bilddaten quartäre Bilddaten abgeleitet werden. Besonders bevorzugt kann der Bildverarbeitungsalgorithmus aus den tertiären Bilddaten quartäre Bilddaten ableiten. Vorzugsweise kann der Bildverarbeitungsalgorithmus den quartären Bilddaten axonale Degenerationsvarianten zuordnen. Axonale Degenerationsvarianten im Sinne der vorliegenden Erfindung umfassen beispielsweise:

i) Instant granular degeneration: Die Degeneration des Axons resultiert in granuläre, voneinander abgetrennte Strukturen (Kerschensteiner, et al. (2005);
ii) Axonal swelling: Die Degeneration des Axons erfolgt durch das Anschwellen bestehender axonaler Spheroide und/oder das Erzeugen neuer axonaler Spheroide und die darauffolgende granuläre Degeneration (Kerschensteiner, et al. (2005); Beirowski, et al. (2005);
iii) Dying-back degeneration: Die Degeneration des Axons, bei der sich das Axon zurückzieht und retrograd degeneriert und die darauffolgende granuläre Degeneration (Beirowski, et al. (2005); und/oder

iv) Transport degeneration: Die Degeneration des Axons, bei der es zu einer erhöhten Transportrate von axonalen Spheroiden innerhalb des Axons kommt in Abwesenheit von Axonal swelling und die darauffolgende granuläre Degeneration (Beirowski, et al. (2005).

Die tertiären und/oder quartären Bilddaten können, insbesondere unter Verwendung des Bildverarbeitungsalgorithmus, als digital aufbereitetes Bild, wie beispielsweise als Fehlfarbenbild oder in einer Heatmap, in Schritt f) erhalten, ausgegeben und/oder dargestellt werden. Vorzugsweise kann in dem digital aufbereiteten Bild erhältlich aus tertiären Bilddaten je nach Änderung der Klassenzugehörigkeit des Pixels jedem Pixel ein bestimmter Farbwert zugeordnet werden und daraus das digital aufbereitete Bild zusammengefügt werden, beispielsweise ein Fehlfarbenbild oder eine Heatmap.

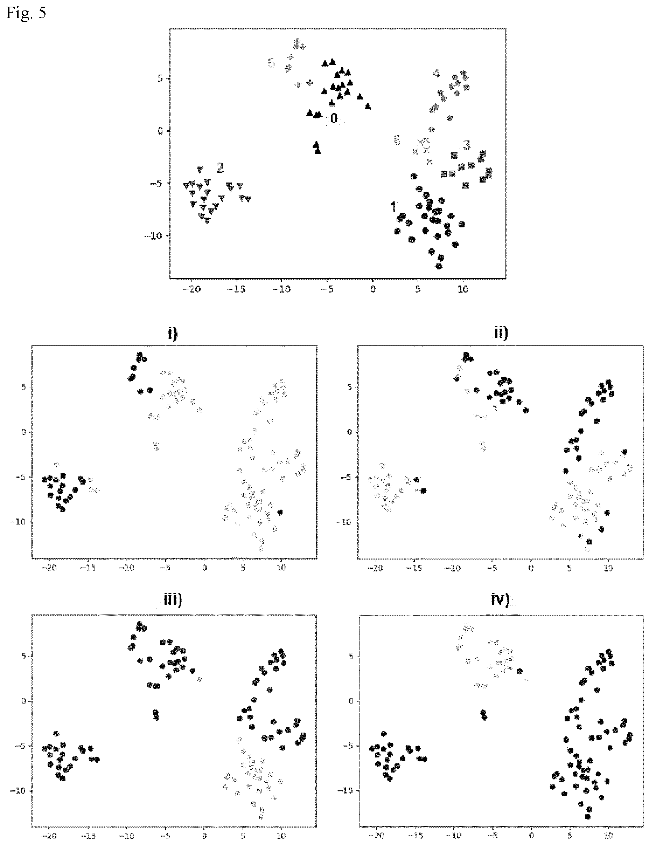

Auch diese Zuordnung und/oder das Zusammenfügen des digital aufbereiteten Bildes kann vorzugsweise unter Verwendung des Bildverarbeitungsalgorithmus durchgeführt werden. Weiterhin können die tertiären Bilddaten beispielsweise als Tabellen und/oder Clusteranalysen ausgegeben werden. Solche Tabellen und/oder Clusteranalysen können insbesondere die Anzahl und/oder Fläche der Pixel umfassen, die eine bestimmte Veränderung der Klassenzugehörigkeit vollzogen haben und/oder den Zeitpunkt, an dem diese Veränderung stattgefunden hat. In Bezug auf die Anzahl der Pixel können insbesondere sogenannte Connected Components Analysen zur Anwendung kommen, die beispielsweise aus Dillencourt et al. (1992) bekannt sind. Weiterhin ist eine Ausgabe der tertiären Bilddaten in Form einer Grafik, wie beispielsweise in Form einer Zeitverlaufskurve, eines Clusterbilds und/oder eines Balkendiagramms und/oder jeder anderen dem Fachmann bekannten Darstellungsweise denkbar. Ein Clusterbild ist beispielhaft in Figur 5 gezeigt. Vorzugsweise kann in dem digital aufbereiteten Bild erhältlich aus quartären Bilddaten je nach Änderung der Klassenzugehörigkeit des Pixels über einen Zeitverlauf dem Pixel ein bestimmter Degenerationstyp zugeordnet werden. Mehr bevorzugt kann der Bildverarbeitungsalgorithmus jeder bestimmten Degenerationvariante einen bestimmten Farbwert zuordnen und daraus das digital aufbereitete Bild zusammenfügen. Weiterhin können die quartären Bilddaten beispielsweise als Tabellen ausgegeben werden; solche Tabellen können insbesondere die Anzahl und/oder Fläche der Pixel umfassen, die eine bestimmte Veränderung der Klassenzugehörigkeit über die Zeit vollzogen haben und/oder einer Degenerationvariante zugeordnet worden sind sowie insbesondere den Zeitpunkt, an dem diese Veränderung stattgefunden hat. Weiterhin ist eine Ausgabe oder ein Erhalten der quartären Bilddaten in Form einer Grafik und/oder jeder anderen dem Fachmann bekannten Darstellungsweise denkbar.

Bevorzugt können die axonalen Degenerationsvarianten anhand der zeitlichen Information und der Veränderung der Klassenzugehörigkeit und/oder dem optischen Fluss auf den Bilddaten bestimmt werden. Diese Bestimmung kann vorzugsweise die Verwendung des Bildverarbeitungsalgorithmus umfassen. Der Begriff "optischer Fluss" kann sich auf das Messen der Bewegung von Pixeln in Videos und/oder mikroskopischen Bilddaten über die Zeit beziehen. Bevorzugt kann für jeden Pixel zu zwei oder mehreren Zeitpunkten ein Vektor erhalten werden. Mehr bevorzugt kann der Vektor die Bewegung des Pixels zwischen den zwei oder mehreren Zeitpunkten bezeichnen.

Für die Klassifizierung der axonalen Degenerationsvarianten kann vorzugsweise ein Encoder-Decoder Recurrent Neural Network (RNN) verwendet werden, wie es beispielsweise aus Bahdanau et al. bekannt ist. Dafür können beispielswiese Bilddaten aus einem Zeitverlaufsexperiments in Form eines Videos mittels des genannten RNN analysiert werden. Ein geeigneter Encoder kann beispielsweise eine gated recurrent unit (GRU) oder eine long short-term memory (LSTM) Einheit sein, die vorzugsweise eine Bildsequenz als Input erhalten kann. Die Bildsequenz kann vorzugswiese vorverarbeitet sein und beispielsweise als eine Abfolge von Histogrammen dargestellt sein.

Als Decoder kann beispielsweise eine weitere GRU oder LSTM in Frage kommen, die vorzugsweise auf Basis eines Kontextvektors trainiert worden sein kann. Der Kontextvektor kann mehr bevorzugt die gewichtete Summe der versteckten Eingangsvektoren und seinen eigenen Ausgaben verwenden, um eine Wahrscheinlichkeit für die Zugehörigkeit zu einer Klasse zu bestimmen. Die Ausgabe des Vektors kann beispielsweise ein C+1 Vektor mit Werten [0,1] sein, wobei C die Anzahl der Klassen repräsentiert. Besonders bevorzugt kann jede Position in dem Vektor die Wahrscheinlichkeit bezeichnen, mit der die Klasse im Eingangsdatensatz, z. B. in einem der Histogramme der Abfolge, bezeichnet war. Im Gegensatz dazu kann die Klasse C+1 vorzugsweise als "nichts vorhanden" bezeichnet werden. Dieser Ausgabevektor kann bevorzugt mit Nullen initialisiert sein.

Das RNN kann vorzugweise mehrere Wiederholungen für die Bestimmung der Klassenzugehörigkeit durchlaufen. Das RNN kann mehr bevorzugt die Zugehörigkeit zu einer Klasse anhand der Bestimmung anderer Klassenzugehörigkeiten entscheiden. Der Kontextvektor im Decoder kann vorzugsweise die gewichtete Summe aller versteckten Vektoren (N-1 hidden vectors) darstellen. Der RNN kann für jede Wiederholung vorzugsweise auf ein anderes Set an versteckten Vektoren zurückgreifen.

Vorzugsweise kann im Anschluss an das Ausgeben und/oder Erhalten automatisch generierter sekundärer Bilddaten zum Zustand der Neurone ein weiterer optionaler Schritt f) zusätzlich das Ausgeben und/oder Erhalten tertiärer und/oder quartärer Bilddaten umfassen.

Insbesondere kann dabei der Zustand der Neurone ausgewählt sein aus: axonalem Wachstum, axonaler Degeneration, axonaler Remodelierung, axonaler Regeneration.

Bevorzugt kann das Verfahren zur Untersuchung von Neuronen vollautomatisch ablaufen. "Vollautomatisch" kann in diesem Zusammenhang heißen, dass lediglich die Präparation der Neuronen manuell erfolgt und die weiteren Schritte dann über einen automatisierten Ablauf, wie beispielsweise eine automatisierte Aussaat und Behandlung der Neuronen in den mikrofluidischen Einheiten mittels eines Pipettierroboters, z.B. BioTek MultiFlo FX Multi-Mode Dispenser und eines dafür passenden Inkubators BioTek BioSpa Automated Incubator, ein automatisiertes Einbringen der mikrofluidischen Einheiten in den Fokus eines Hochdurchsatz-Mikroskops und/oder ein automatisierter Ablauf des Generierens der mikroskopischen Bilddaten beispielswiese unter Verwendung eines automatisierten Mikroskopiertischs. Weiterhin kann die Aussaat, und/oder die Kultivierung der Neuronen vorzugsweise unter der Verwendung von Mehrkanalpipetten parallelisiert ablaufen kann. Besonders bevorzugt läuft auch das Ausgeben und/oder Erhalten der sekundären und tertiären Bilddaten automatisch ab.

Ferner bezieht sich die vorliegende Erfindung auf ein System zur automatischen Untersuchung von Neuronen. Das System umfasst mindestens eine Testvorrichtung gemäß der vorliegenden Erfindung, mindestens ein Mikroskop, und mindestens eine Auswerteeinheit, beispielweise einen Computer, eine Computing Cloud, ein Netzwerk, einen Tabletcomputer und/oder ein Smartphone. Vorzugsweise ist die Auswerteeinheit geeignet ein Python-Skript auszuführen. Die Auswerteeinheit ist vorzugsweise geeignet das Verfahren zur Untersuchung von Neuronen gemäß der vorliegenden Erfindung auszuführen; weiterhin vorzugsweise führt die Auswerteeinheit das Verfahren zur Untersuchung von Neuronen gemäß der vorliegenden Erfindung aus.

Ferner wird im Rahmen der vorliegenden Erfindung ein Computerprogramm vorgeschlagen, das bei Ablauf auf einem Computer oder Computer-Netzwerk das erfindungsgemäße Verfahren in einer seiner Ausgestaltungen ausführt.

Weiterhin wird im Rahmen der vorliegenden Erfindung ein Computerprogramm mit Programmcode-Mitteln vorgeschlagen, um das erfindungsgemäße Verfahren in einer seiner Ausgestaltungen durchzuführen, wenn das Programm auf einem Computer oder Computer-Netzwerk ausgeführt wird. Insbesondere können die Programmcode-Mittel auf einem computerlesbaren Datenträger und/oder einem computerlesbaren Speichermedium gespeichert sein.

Die Begriffe "computerlesbarer Datenträger" und "computerlesbares Speichermedium", wie sie hier verwendet werden, können sich insbesondere auf nicht-transitorische Datenspeicher beziehen, beispielsweise ein Hardware-Datenspeichermedium, auf welchem computerausführbare Instruktionen gespeichert sind. Der computerlesbare Datenträger oder das computerlesbare Speichermedium können insbesondere ein Speichermedium wie ein Random-Access Memory (RAM) und/oder ein Read-Only Memory (ROM) sein oder umfassen.

Außerdem wird im Rahmen der vorliegenden Erfindung ein Datenträger vorgeschlagen, auf dem eine Datenstruktur gespeichert ist, die nach einem Laden in einen Arbeits- und/oder Hauptspeicher eines Computers oder Computer-Netzwerkes das erfindungsgemäße Verfahren in einer seiner Ausgestaltungen ausführen kann.

Auch wird im Rahmen der vorliegenden Erfindung ein Computerprogramm-Produkt mit auf einem maschinenlesbaren Träger gespeicherten Programmcode-Mitteln bereitgestellt, um das erfindungsgemäße Verfahren in einer seiner Ausgestaltungen durchzuführen, wenn das Programm auf einem Computer oder Computer-Netzwerk ausgeführt wird.

Dabei wird unter einem Computer-Programmprodukt das Programm als handelbares Produkt verstanden. Es kann grundsätzlich in beliebiger Form vorliegen, so zum Beispiel auf Papier oder einem computerlesbaren Datenträger und kann insbesondere über ein Datenübertragungsnetz verteilt werden.

Schließlich wird im Rahmen der vorliegenden Erfindung ein moduliertes Datensignal vorgeschlagen, welches von einem Computersystem oder Computernetzwerk ausführbare Instruktionen zum Ausführen eines Verfahrens nach einer der beschriebenen Ausführungsformen enthält.

Im Hinblick auf die computer-implementierten Aspekte der Erfindung können einer, mehrere oder sogar alle Verfahrensschritte des Verfahrens gemäß einer oder mehreren der hier vorgeschlagenen Ausgestaltungen mittels eines Computers oder Computer-Netzwerks durchgeführt werden. Somit können, allgemein, jegliche der Verfahrensschritte, einschließlich der Bereitstellung und/oder Manipulation von Daten mittels eines Computers oder Computer-Netzwerks durchgeführt werden. Allgemein können diese Schritte jegliche der Verfahrensschritte umfassen, ausgenommen der Schritte, welche manuelle Arbeit erfordern, beispielsweise das Bereitstellen von Proben und/oder bestimmte Aspekte der Durchführung tatsächlicher Messungen.

Besondere Aspekte der Erfindung betreffen:

Aspekt 1. Mikrofluidische Testvorrichtung zur Kultivierung und/oder Untersuchung von Neuronen, vorzugsweise von Axonen, umfassend mindestens eine Trägerplatte und einen Körper, wobei der Körper eine Mehrzahl an mikrofluidische Einheiten aufweist, wobei jede mikrofluidische Einheit mindestens zwei sich gegenüberliegende Kammern umfasst, dadurch gekennzeichnet, dass der Körper monolithisch ausgestaltet ist.

Aspekt 2. Mikrofluidische Testvorrichtung nach Aspekt 1, wobei der Körper mindestens 6, vorzugsweise mindestens 12 oder mindestens 16 mikrofluidische Einheiten aufweist.

Aspekt 2. Mikrofluidische Testvorrichtung nach Aspekt 1 oder 2, dadurch gekennzeichnet, dass die mindestens zwei sich gegenüberliegenden Kammern jeweils ein Kompartiment aufweist, das sich in Richtung der anderen Kammer erstreckt.

Aspekt 3. Mikrofluidische Testvorrichtung nach Aspekt 1 oder 2, dadurch gekennzeichnet, dass die Kompartimente Mikrokanäle aufweisen.

Aspekt 4. Mikrofluidische Testvorrichtung nach einem der Aspekte 1 bis 3, dadurch gekennzeichnet, dass die Mikrokanäle eine Verbindung zwischen den beiden Kompartimenten darstellen.

Aspekt 5. Mikrofluidische Testvorrichtung nach einem der Aspekte 1 bis 4, dadurch gekennzeichnet, dass die Mikrokanäle eine maximale Breite von 10 $\mu$m und/oder eine maximale Höhe von 5 $\mu$m und/oder eine maximale Länge von 4350 $\mu$m aufweisen.

Aspekt 6. Mikrofluidische Testvorrichtung nach einem der Aspekte 1 bis 5, dadurch gekennzeichnet, dass die mindestens zwei sich gegenüberliegende Kammern einen Durchmesser von 1 bis 10 mm und/oder eine maximale Höhe von 15 mm aufweisen.

Aspekt 7. Mikrofluidische Testvorrichtung nach einem der Aspekte 2 bis 6, dadurch gekennzeichnet, dass die Kompartimente mit der jeweiligen Kammer direkt verbunden sind.

Aspekt 8. Mikrofluidische Testvorrichtung nach einem der Aspekte 1 bis 7, dadurch gekennzeichnet, dass die Mikrokanäle einen Maximalabstand zueinander von maximal 100 $\mu$m aufweisen, bevorzugt von maximal 75 $\mu$m, mehr bevorzugt von maximal 50 $\mu$m.

Aspekt 9. Mikrofluidische Testvorrichtung nach einem der Aspekte 2 bis 8, dadurch gekennzeichnet, dass die Kompartimente über maximal 250 Mikrokanäle miteinander verbunden sind.

Aspekt 10. Mikrofluidische Testvorrichtung nach einem der Aspekte 1 bis 9, dadurch gekennzeichnet, dass die Testvorrichtung mindestens eines der folgenden Materialien umfasst oder daraus besteht: Glas, wie beispielsweise Quarzglas, Bleiglas, Kalknatronglas, Borsilikaglas, Silicon, Quarz, Keramik; ein Polymer auf Siliziumbasis, beispielsweise Polydimethylsiloxan (PDMS); Elastomere, beispielsweise Thermoset Polyester und thermoplastische Polymer wie Polystyrol, Polycarbonat, Polyoxymethylen, Polynmethyl methacrylate (PMMA), Polyethyleneglycol-Diacrylat, Polyethylenterephthalat, Teflon, Perfluoroalkoxy-Polymere, Tetrafluorethylen-Hexafluorpropylen-Copolymer, Polyfluoropolyetherdiol-Methacrylat, Polyurethan; zusammengesetzte Materialeien wie zyklische Olefin-Polymere, eine Mischung aus PMMA)/PDMS, eine Mischung aus Papier/PDMS; Papier; und/oder Hydrogele.

Aspekt 11. Mikrofluidische Testvorrichtung nach einem der Aspekte 1 bis 10, dadurch gekennzeichnet, dass die Testvorrichtung einen Boden aufweist, der die Kompartimente und/oder die Kammern auf der Unterseite verschließt, vorzugsweise verschließt der Boden, die Kammern, die Kompartimente und die Mikrokanäle auf der Unterseite.

Aspekt 12. Mikrofluidische Testvorrichtung nach einem der Aspekte 2 bis 11, dadurch gekennzeichnet, dass die Kompartimente und/oder die Mikrokanäle eine Überdeckung aufweisen.

Aspekt 13. Mikrofluidische Testvorrichtung nach einem der Aspekte 1 bis 12, dadurch gekennzeichnet, dass der Abstand der gegenüberliegenden Kammern zwischen 2 und 10 mm beträgt.

Aspekt 14. Mikrofluidische Testvorrichtung nach einem der Aspekte 1 bis 13, dadurch gekennzeichnet, dass der Boden der Kammern, der Kompartimente und der Mikrokanäle auf derselben Ebene liegen.

Aspekt 15. Mikrofluidische Testvorrichtung nach einem der Aspekte 1 bis 14, dadurch gekennzeichnet, dass der Boden der Testvorrichtung von der Trägerplatte, vorzugsweise von einer Trägerplatte umfassend oder bestehend aus mindestens einem Material ausgewählt aus Glas und/oder Kunststoff gebildet wird; vorzugsweise ausgewählt aus: Silicon; Glas, wie beispielsweise Quarzglas, Bleiglas, Kalknatronglas, Borsilikaglas; Quarz; Keramik; Elastomere, wie beispielsweise PDMS, Thermoset Polyester, thermoplastische Polymere wie Polystyrol, Polycarbonat, Polyoxymethylen, Polyethylen, Polymethylmethacrylate, Polyethylenglycol-Diacrylat, Polyethylenterephthalat, mit Glycol modifiziertes Polytetrafluorethylen, Polyurethan, gebildet wird.

Aspekt 16. Verfahren zur Untersuchung von Neuronen, umfassend die folgenden Schritte:

a) Generieren mikroskopischer Bilddaten von Neuronen in mindestens einer mikrofluidischen Einheit, wobei die Neuronen jeweils in einer mikrofluidischen Einheit vorliegen, vorzugsweise in einer Testvorrichtung nach einem der Ansprüche 1 bis 15 vorliegen, wobei von einer mikrofluidischen Einheit mindestens ein mikroskopisches Bild erzeugt wird, wobei jedes mikroskopische Bild eine Mehrzahl an Pixeln aufweist;

b) Generieren von sekundären Bilddaten unter Verwendung eines Bildverarbeitungsalgorithmus, wobei der Bildverarbeitungsalgorithmus für jedes Pixel der in Schritt a) generierten Bilder mindestens eine Klassenzugehörigkeit bestimmt; bevorzugt unter Verwendung eines Machine-Learning-Algorithmus, mehr bevorzugt unter Verwendung eines Deep Learning Algorithmus; welcher anhand von mikroskopischen Bilddaten mit bekannten Informationen trainiert wurde;

c) Ausgeben und/oder Erhalten automatisch generierter sekundärer Bilddaten zum Zustand der Neurone; und optional

cc) Erzeugen von Daten zum Zustand der Neurone aus den sekundären Bilddaten; und optional;

d) Zusammenfügen eines digital aufbereiteten Bildes basierend auf den sekundären Bilddaten;

e) Darstellen der automatisch generierten sekundärer Bilddaten oder des digital aufbereiteten Bildes auf einer Bildwiedergabevorrichtung; und/oder

f) Ausgeben und/oder Erhalten automatisch generierter tertiärer und/oder quartärer Bilddaten.

Aspekt 17. Verfahren zur Untersuchung von Neuronen nach Aspekt 16, wobei das Generieren der mikroskopischen Bilddaten in Schritt a) das Aufnehmen von mikroskopischen Bilddaten mittels eines Mikroskops umfasst, bevorzugt Daten von lichtmikroskopischen Aufnahmen und/oder fluoreszenzmikroskopischen Aufnahmen; mehr bevorzugt Daten von Phasenkontrastaufnahmen umfasst.

Aspekt 18. Verfahren zur Untersuchung von Neuronen nach Aspekt 16 oder 17, wobei die Bilddaten in Schritt a) und b) mikroskopische Bilddaten von Axonen umfassen.

Aspekt 19. Verfahren zur Untersuchung von Neuronen nach einem der Aspekte 16 bis 18, wobei der Bildverarbeitungsalgorithmus in Schritt b) für jedes Pixel der in Schritt a) generierten Bilddaten mindestens eine Klassenzugehörigkeit bestimmt; vorzugsweise mindestens die Zugehörigkeit zu einer der folgenden Klassen bestimmt: Hintergrund, Axon, axonaler Spheroid, axonales Fragment.

Aspekt 20. Verfahren zur Untersuchung von Neuronen nach einem der Aspekte 16 bis 19, wobei das Generieren von sekundären Bilddaten in Schritt b) mit einem Convolutional Neural Network (CNN) stattfindet; insbesondere unter Verwendung eines U-Net; beispielsweise kann ein U-Net mit VGG16-Encoder verwendet werden.

Aspekt 21. Verfahren zur Untersuchung von Neuronen nach einem der Aspekte 16 bis 20, wobei das Generieren von sekundären Bilddaten in Schritt b) das Segmentieren der Bilddaten umfasst.

Aspekt 22. Verfahren zur Untersuchung von Neuronen nach einem der Aspekte 16 bis 21, wobei das Zusammenfügen des digital aufbereiteten Bildes in Schritt d) basierend auf tertiären Bilddaten abgeleitet aus den sekundären Bilddaten stattfindet.

Aspekt 23. Verfahren zur Untersuchung von Neuronen nach einem der Aspekte 16 bis 22, wobei das Ausgeben und/oder Erhalten automatisch generierter sekundärer Bilddaten zum Zustand der Neurone zusätzlich das Erhalten und/oder Ausgeben tertiärer und/oder quartärer Bilddaten umfasst; wobei vorzugsweise der Zustand der Neurone ausgewählt ist aus: axonalem Wachstum, axonaler Degeneration, axonaler Remodelierung, axonaler Regeneration.

Aspekt 24. Verfahren zur Untersuchung von Neuronen nach einem der Aspekte 16 bis 23, wobei die sekundären Bilddaten automatisch generierte sekundäre Bilddaten zum Zustand der Neurone umfassen, wobei diese vorzugsweise in Form einer graphischen Darstellung und/oder in Form einer Tabelle vorliegen.

Aspekt 25. Verfahren zur Untersuchung von Neuronen nach einem der Aspekte 16 bis 24, wobei der Bildverarbeitungsalgorithmus mit mindestens 15 Trainingsproben, vorzugsweise mit mindestens 30 Trainingsproben, trainiert wurde; wobei vorzugsweise jede Trainingsprobe mindestens je ein Bild umfasst, welches aus Pixeln besteht oder diese umfasst und wobei zu jedem einzelnen Pixel mindestens Informationen über eine Klassenzugehörigkeit bekannt sind.

Aspekt 26. System zur automatischen Untersuchung von Neuronen umfassend mindestens eine Testvorrichtung einem der Aspekte 1 bis 15, mindestens ein Mikroskop, mindestens eine Auswerteeinheit, beispielweise einen Computer, eine Computing Cloud, ein Netzwerk, einen Tabletcomputer und/oder ein Smartphone; wobei die Auswerteeinheit vorzugsweise geeignet ist das Verfahren nach einem der Aspekte 16 bis 25 auszuführen, vorzugsweise ist die Auswerteeinheit geeignet ein Python-Skript auszuführen.

Aspekt 27. Verfahren zur Kultivierung von Neuronen umfassend:

a) Bereitstellen einer mikrofluidischen Testvorrichtung nach einem der Aspekte 1 bis 15,
b) Aussäen von Neuronen in mindestens eine der Kammern und/oder Kompartimente, so dass mindestens eine angesäte Kammer entsteht,
c) Inkontaktbringen zumindest eines Teils der angesäten Kammern mit einem Kulturmedium,
d) Kultivieren der Neuronen.

Aspekt 28. Verfahren nach Aspekt 27, weiterhin umfassend:
e) Co-Kultivieren von weiteren Zellen, beispielsweise Gliazellen, Muskelzellen, Endothelzellen, Drüsenzellen, Darmzellen, Tumorzellen.

Der Inhalt sämtlicher hierin zitierten Literaturstellen ist hiermit durch Bezugnahme auf den jeweiligen speziellen Offenbarungsgehalt und in ihrer Gesamtheit aufgenommen.

FIGUREN

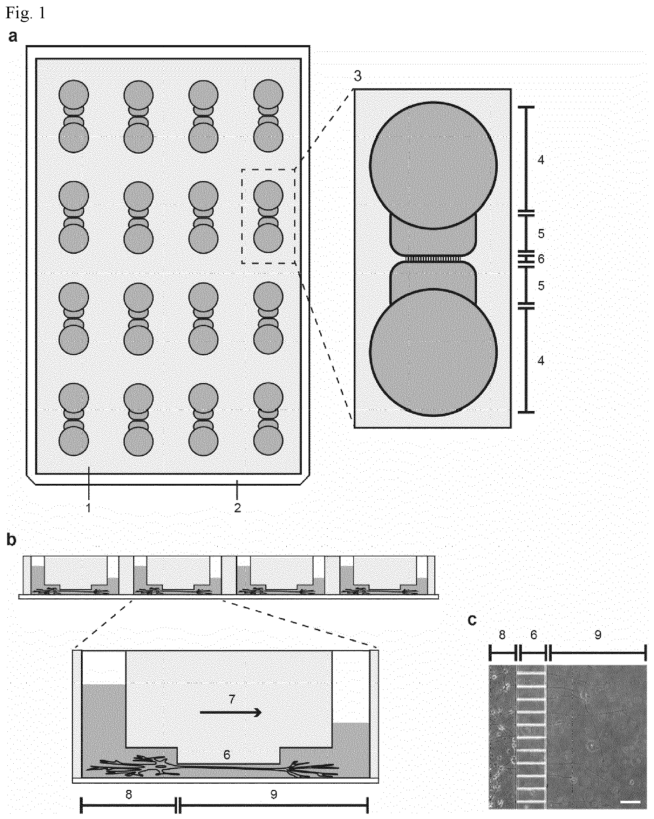

**Fig. 1. Mikrofluidische Testvorrichtung zur Untersuchung von Neuronen**. **a**, Der Körper der Testvorrichtung (1) ist auf einer Trägerplatte (2) befestigt und beinhaltet 16 mikrofluidische Einheiten. Eine mikrofluidische Einheit (3) besteht aus zwei gegenüberliegenden Kammern (4), die über Kompartimente (5) und Mikrokanäle (6) miteinander verbunden sind. **b**, Neurone werden in ein erstes der beiden Kompartimente ausgesät (Seite der Zellkörper und Dendrite (8)) und ihre Axone (9) wachsen durch die Mikrokanäle (6) in das zweite Kompartiment. Dies erfolgt entlang eines mikrofluidischen Flusses (7), der durch einen Unterschied im Flüssigkeitsstand der beiden Kammern hergestellt wird. **c**, Phasenkontrastaufnahme der Neurone in der Testvorrichtung und der räumlichen Trennung von Zellkörpern und Axonen. Messbalken = 100 $\mu$m.

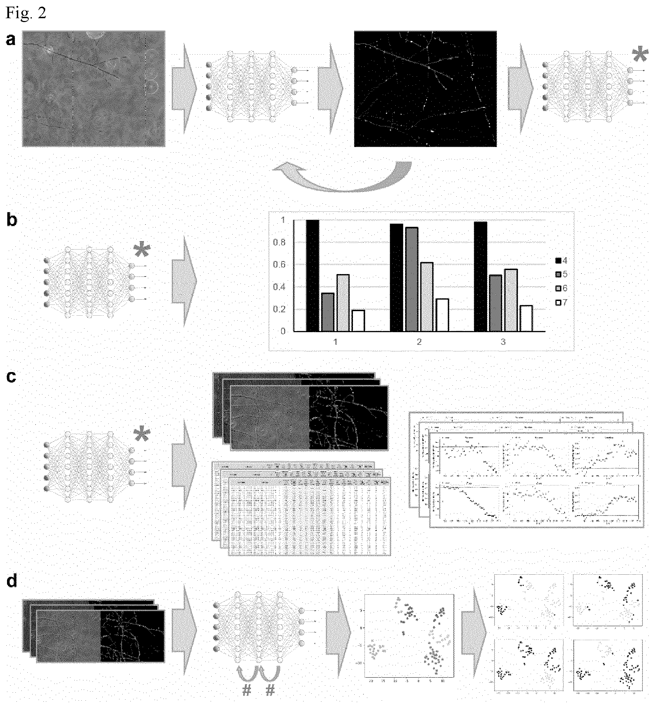

**Fig. 2. Verfahren zur automatisierten Untersuchung von Neuronen**. **a**, Für das Training wurden die die Trainingsproben/Bilddatensätze wurden manuell gelabelt, wobei jedem Pixel eine von vier Klassen zugeordnet wurde: Hintergrund, Axon, axonaler Spheroid und axonales Fragment. Das convolutional neural network lernte dann diese Klassenzuordnung und passte die Gewichte zwischen einzelnen Neuronen der versteckten Ebenen nach jeder Epoche entsprechend an. **b**, Das trainierte convolutional neural network wurde dann mit einem unabhängigen, vorher nicht gesehen, Validierungsdatensatz validiert. Der Graph zeigt die Precision (1, Ratio aus richtig positiven Zuordnungen geteilt durch die Summe aus richtig positiven und falsch positiven Zuordnungen, d.h. einer hohen Precision liegt eine niedrige falsch positiv Rate zugrunde), Recall (2, Ratio aus richtig positiven Zuordnungen geteilt durch die Summe aus richtig positiven und falsch negativen Zuordnungen, entspricht der Sensitivität der Klassenzuordnung) und den F1 Score (3, das harmonische Mittel aus Precision und Recall, berechnet als 2 * (Recall * Precision) / (Recall + Precision) bzw. als 2*TPs / (2*TPs + FP + FN)) der Klassen Hintergrund (4), Axon (5), axonalem Spheroid (6) und Fragment (7). **c**. Das trainierte convolutional neural network kann dann angewendet werden und erzeugt sekundäre (segmentierte Bilddatensätze) und tertiäre (Zeitverlauf der Pixelverteilung) Bilddaten. **d**, Die erzeugten tertiären Bilddaten wurden für das Training eines rekurrenten Neuronalen Netzes genutzt, welches mittels Rückkopplungen (#) quartäre Bilddaten (Varianten axonaler Degeneration) erzeugt.

**Fig. 3. Das Verfahren zur automatisierten Untersuchung von Nervenfortsätzen erkennt axonales Wachstum, Remodelierung und Degeneration.** Exemplarische Phasenkontrast-Bilder (linke Spalte) und die vom CNN generierten dazugehörigen Maskenbilder (rechte Spalte). Auf den Maskenbildern ist der Hintergrund in schwarz, Axone in dunkelgrau, axonale Spheroide in mittelgrau und Fragmente in hellgrau dargestellt. Es werden axonales Wachstum (x), axonale Remodelierung (y) und axonale Degeneration (z) erkannt. Messbalken = 50 μm.

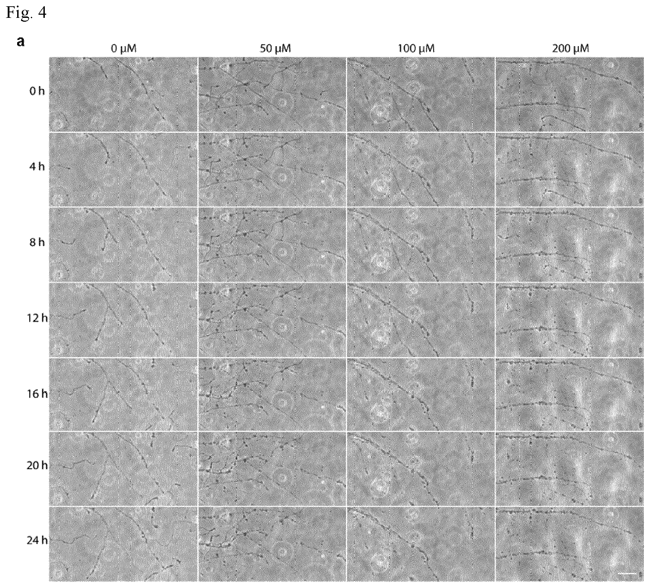

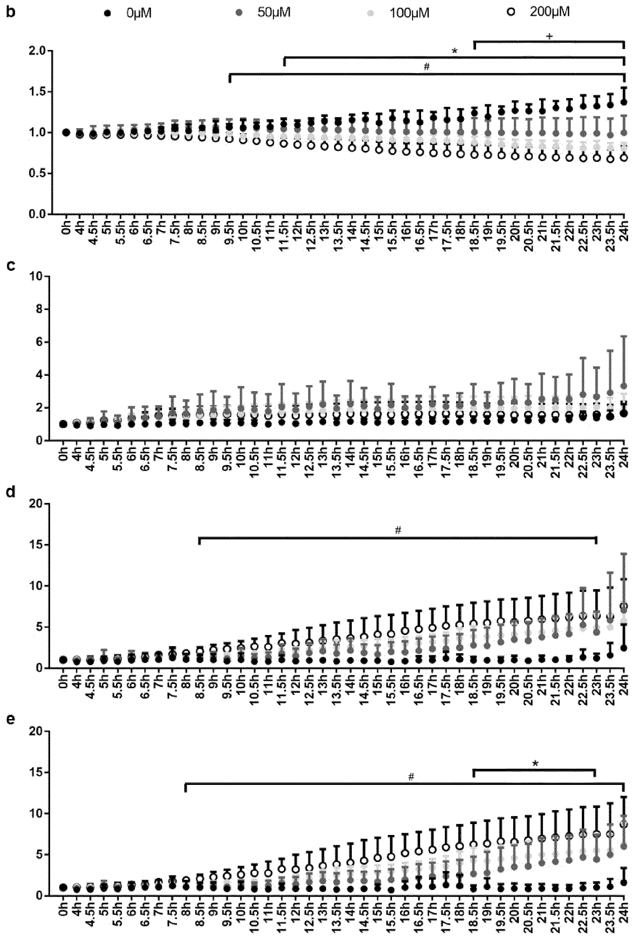

**Fig. 4. Das Verfahren zur automatisierten Untersuchung von Neuronen erkennt zeit- und konzentrationsabhängige axonale Degeneration in einem experimentellen Modell für hämorrhagischen Schlaganfall. a**, Axone, die über 24 Stunden mit Hämin behandelt wurden (50, 100, 200 μM), weisen morphologische Veränderungen im Vergleich zu Vehikel-behandelten Axonen (0 μM) auf, die weiter wuchsen. Messbalken = 50 μm. **b**, Die Analyse der Phasenkontrast-Bilder über 24 Stunden zeigte, dass die Axonfläche im Vergleich zur Kontrolle (0 μM) ab 9,5h (200 μM), 11,5h (100 μM) und 18,5h (50 μM) signifikant kleiner war. **c**, Bei der Fläche der axonalen Spheroide ergaben sich keine Unterschiede zwischen den Konzentrationen. **d**, Die Fläche der Fragmente war zwischen 8,5h und 23h (200 μM vs. 0 μM) signifikant erhöht ebenso wie **e**, der axonale Degenerationsindex erhöht war zwischen 8h und 24h (200 μM vs. 0 μM) und zwischen 18,5h und 23h (100 μM vs. 0 μM). Die x-Achse zeigt die Zeit, die y-Achse die normalisierte Fläche im Vergleich zum Zeitpunkt 0h (**b-d**) bzw. den axonalen Degenerationsindex (**e**). n=6 unabhängige Neuronenpräparationen, p-Werte: +, *, # < 0.05; + = 50 μM vs. 0 μM, * = 100 μM vs. 0 μM, # = 200 μM vs. 0 μM.

**Fig. 5. Das Verfahren zur Untersuchung von Neuronen erkennt unterschiedliche Varianten der axonalen Degeneration.** Es wurden 7 verschiedene Cluster identifiziert, die die vier Varianten der axonalen Degeneration i) Instant granular degeneration, ii) Axonal swelling, iii) Dying-back degeneration und iv) Transport degeneration beschreiben. Graue Punkte gehören zur jeweiligen Variante der axonalen Degeneration, während schwarze Punkte nicht dazu gehören.

BEISPIELE

Die nachfolgenden Beispiele dienen der Illustration der Erfindung. Sie dürfen im Hinblick auf den Schutzumfang nicht in einschränkender Weise ausgelegt werden.

**Beispiel 1: Erstellen der mikrofluidischen Testvorrichtung**

Polydimethylsiloxane (PDMS, Biesterfeld) wurde im Verhältnis 1:10 gemischt und Vakuum wurde 30 Minuten angelegt (-0.006 Pa in einem Vakuumexsikkator, Jeio Tech). PDMS wurde in die Negativ-Gussform aus Epoxid gegossen und erneut Vakuum 30 Minuten angelegt. Das flüssige PDMS wurde bei 73 °C ausgehärtet, um die Polymerisierung des PDMS einzuleiten. Die mikrofluidische Testvorrichtung wurde aus der Gussform entnommen und die Kammern mit einer 8-mm Biopsiestanze (DocCheck Shop GmbH) gestanzt. Glasplatten wurden mittels Ultraschallbehandlung (Sonicator Elmasonic S, Elma Schmidbauer GmbH) gereinigt und anschließend mit Äthanol gewaschen. Es erfolgte eine Plasmabehandlung (Harrick Plasma) 2 Minuten auf der höchsten Stufe, um die Silanolgruppen zwischen Glasplatte und mikrofluidischer Testvorrichtung zu aktivieren und so eine beständige Anheftung zu gewährleisten. Eine beispielhafte Testvorrichtung mit 16 mikrofluidischen Einheiten wurde erhalten. Die beispielhafte Testvorrichtung hat die folgenden Ausmaße: Länge 115 mm, Breite 73,5 mm; Höhe 14,4 mm. Dabei haben die Kammern einen Durchmesser von 8 mm. Der Abstand zwischen den sich gegenüberliegenden Kammern beträgt 4,5 mm; die Kompartimente haben eine Breite von 4,58 mm; die Mikrokanäle haben eine Länge von 150 μm.

Die Kammern, Kompartimente und Mikrokanäle der Testvorrichtung wurden anschließend mit Äthanol sowie zweimal mit destilliertem Wasser gewaschen, um Schmutz zu entfernen. Das destillierte Wasser wurden von den Kammern abgesaugt und es erfolgte eine Beschichtung mit 0,1 mg/mL Poly-D-Lysin (PDL, Sigma Aldrich) in 0,02 M Boratpuffer (0,25 % Boratsaeure, 0,38 % Natriumtetraborat (Merck) in destilliertem Wasser, pH 8,5) bei 4 °C über Nacht. Das PDL wurde am nächsten Morgen aus den Kammern abgesaugt und es erfolgt eine Beschichtung mit 50 μg/mL Laminin (Sigma Aldrich) bei 4 °C über Nacht. Am Tag der Isolation der Neuronen wurden die Kammern, Kompartimente und Mikrokanäle der Testvorrichtung nach Absaugen des Laminins zweimal mit erwärmten Medium gewaschen. Unmittelbar vor der Aussaat wurde das Medium aus den Kammern entfernt.

## Beispiel 2: Gewinnung und Kultivierung der Neurone in der Testvorrichtung

Primäre kortikale Neurone (PCNs) wurden aus E14 Embryonen der Maus gewonnen. Während der Isolation wurden die Gehirne in PBS auf Eis gelagert. Die kortikalen Hemisphären wurden aus dem PBS in $CO_2$-gesättigte Papainlösung überführt (0,33% Papain (Carl Roth), 0,017 % L-Cysteine (Sigma-Aldrich), 0,1 % 0.5 M EDTA (Carl Roth), 1 % Natriumacetat (Carl Roth) und 30 mL EBSS (10 % Earle's Balanced Salt Solution (Sigma Aldrich) und 0,22 % Natriumbicarbonat (Möller Chemie), pH 7.3)) und 20 Minuten bei 37 °C inkubiert. Das Gewebe wurde mechanisch mittels serologischen Pipetten aufgebrochen und weitere 3 Minuten bei 37 °C inkubiert. Dann wurde 1 mg/mL DNAse (Sigma Aldrich) hinzugefügt und die Zellsuspension bei 250 x g 5 Minuten zentrifugiert. Nach der Entfernung des Überstandes wurde das Zellpellet in 5 mL EBSS (1%) + bovinem Serumalbumin (BSA)/Trypsin Inhibitor resuspendiert (1% BSA (Sigma Aldrich) und 1% Trypsin Inhibitor Chicken Egg White (Sigma Aldrich) in 50 mL EBSS) und weitere 5 mL BSA/Trypsin Inhibitor wurden vorsichtig hinzugefügt ohne die Zellsuspension zu verwirbeln. Die Suspension wurde dann bei 250 x g 10 Minuten zentrifugiert, der Überstand wurde abgesaugt und das Pellet in 5 mL MEM+Glutamax Medium (Thermo Fisher Scientific) zzgl. 10 % fetales Kälberserum (Thermo Fisher Scientific), 1% Pferdeserum (GE Health Life Sciences) und 1 % Penicillin/Streptomycin (Biochrom) resuspendiert. Die Zellsuspension wurde dann mittel 70 μm Zellsieb (VWR) gefiltert.

Primäre kortikale Neurone wurden mit einer Dichte von 6000 Zellen pro mm$^2$ Kompartimentfläche in die mikrofluidischen Einheiten ausgesät und adhärierten innerhalb von 30 Minuten bei 37 °C. Zur Herstellung des mikrofluidischen Flusses wurden dann 150 μL Medium in das erste Kompartiment mit Zellkörpern hinzugefügt und 100 μL in das axonale Kompartiment. Die Neurone wurden bei 37 °C, 95% Luftfeuchtigkeit und 5 % $CO_2$ kultiviert. Das MEM+Glutamax Medium wurde durch Neurobasal Plus Medium zzgl. 2 % B-27 Plus Supplement, 1 % Natriumpyruvat (alles Thermo Fisher Scientific) und 1 % Penicillin/Streptomycin (Biochrom) am ersten Tag in Kultur ersetzt. Die Volumenunterschiede des Mediums zwischen den Kammern wurden beibehalten, um den mikrofluidischen Fluss für das gerichtete Wachstum der Axone weiterhin zu ermöglichen. Nach 4 bis 5 Tagen in Kultur sind die ersten Axone im axonalen Kompartiment sichtbar und wachsen weiterhin innerhalb der nächsten Tage und Wochen. Durch einen wöchentlichen Mediumwechsel ist eine Langzeitkultur von 1-2 Monaten möglich.

## Beispiel 3: Trainieren des Bildverarbeitungsalgorithmus

Für das Training der Segmentierung der Klassen mittels CNN (**Fig. 2a**) wurden 33 Bilder ausgewählt und die dazugehörige Masken durch manuelles Labeln mittels GIMP erstellt. In jeder Maske wurde jedem Pixel der Wert 0, 1, 2 oder 3 zugeordnet, je nachdem welcher Klasse der Pixel angehörte, wobei 0 der Klasse Hintergrund, 1 der Klasse Axon, 2 der Klasse axonales Spheroid und 3 der Klasse axonales Fragment entsprach. Für das Training wurde eine Variante der U-net Architektur (Ronneberger et al.) verwendet und das Vgg16 Netzwerk (Simonyan et al.) wurde als Encoder verwendet. Das Netzwerk wurde mit 120 Epochen und dem Adam Optimizer, einer Batchgröße von 2 und einer Lernrate von 0,01 trainiert. Die Input-Bilder wurden mittels Mittelwert und Standardabweichung des Imagenet (Deng et al.) standardisiert.

## Beispiel 4: Validieren des Bildverarbeitungsalgorithmus

Zur Validierung des Trainingssets/Bildverarbeitungsalgorithmus wurde ein unabhängiges Validierungsset, welches das CNN noch nicht gesehen hatte, generiert. Dafür wurden ebenfalls Masken durch manuelles Labeln erstellt, die die Grundwahrheit darstellen. Der Output des CNN wurde mittels Precision, Recall und F1 Score mit der Grundwahrheit für jede Klasse separat verglichen. Dabei wurde die Leistung des CNN berechnet, indem die F1 Scores aller Validierungsbilder pro Klasse gemittelt wurden (**Fig. 2c**). Der F1 Score ist definiert als 2* richtig Positive / (2* richtig Positive + falsch Positive + falsch Negative)

## Beispiel 5: Automatisierte Untersuchung axonaler Degeneration über die Zeit

Das axonale Kompartiment wurde mit 0 (Vehikel), 50, 100 und 200 μM Hämin (Sigma Aldrich) behandelt, wobei das Volumen des Mediums zwischen den zwei Kammern ausgeglichen wurde, um den mikrofluidischen Fluss zu unterbinden. Hämin ist oxidiertes Häm und wird verwendet um Schaden durch Blutungen im Gehirn in Zellkultur zu simulieren (Zille et al., 2017). Die Testvorrichtung wurde dann mittels Zeitraffermikroskopie (Olympus IX81) über 24 Stunden mit einem Zeitintervall von 30 Minuten beobachtet, wobei die Kulturbedingungen konstant gehalten wurden (37 °C in einer feuchten Atmosphäre bei 5 % $CO_2$).

Alle Formen des Zustands der Neurone, wie axonales Wachstum, axonale Degeneration, axonales Remodelierung und axonale Regeneration konnte vom CNN identifiziert werden (**Fig. 3**). Während die Vehikelbehandelten Axone über den 24-stündigen Verlauf weiter wuchsen, löste Hämin eine konzentrations- und zeitabhängige Degeneration der Axone aus (**Fig. 4**). Die axonale Fläche war dabei signifikant kleiner ab 9,5 Stunden (200 $\mu$M), 11, 5h (100 $\mu$M), und 18,5h (50 $\mu$M) nach Häminbehandlung im Vergleich zur Kontrolle (0 $\mu$M). Die Fragmentfläche war signifikant erhöht zwischen 8,5 und 23 Stunden (200 $\mu$M vs. 0 $\mu$M), während der axonale Degeneratonsindex sich zwischen 8 und 24 Stunden (200 $\mu$M vs. 0 $\mu$M) bzw. zwischen 18,5 und 23 Stunden (100 $\mu$M vs. 0 $\mu$M) erhöhte. Die Fläche der axonalen Spheroide blieb hingegen unverändert.

**Beispiel 6: Erkennung von Varianten axonaler Degeneration**

Zur Erkennung der Varianten axonaler Degeneration wurde ein RNN trainiert (**Fig. 2d**). Dafür wurden Zeitverläufe der vier Varianten axonaler Degeneration genutzt: i) Instant granular degeneration, ii) Axonal swelling, iii) Dying-back degeneration und iv) Transport degeneration. Das RNN wurde trainiert, die Veränderung der Klassenzugehörigkeit zu nutzen. Das RNN identifizierte 7 Cluster, deren unterschiedliche Kombination, die Zugehörigkeit zu einer der vier Varianten axonaler Degeneration vorhersagt (**Fig. 5**).

Literatur

Bahdanau et al. (2015) "Neural machine translation by jointly learning to align and translate," in 3rd International Conference on Learning Representations, ICLR 2015 - Conference Track Proceedings.

Beirowski, et al. (2005) "The Progressive Nature of Wallerian Degeneration in Wild-Type and Slow Wallerian Degeneration (WldS) Nerves." BMC Neuroscience 6 (1): 6.

Deng, J., Dong, W., Socher, R., Li, L. J., Li, K., & Fei-Fei, L. (2009, June). Imagenet: A large-scale hierarchical image database. In 2009 IEEE conference on Computer vision and pattern recognition (pp. 248-255).

Dillencourt et al. (1992). "A general approach to connected-component labeling for arbitrary image representations". Journal of the ACM. 39 (2): 253

Kerschensteiner, Martin, Martin E Schwab, Jeff W Lichtman, and Thomas Misgeld. 2005. "In Vivo Imaging of Axonal Degeneration and Regeneration in the Injured Spinal Cord." Nature Medicine 11 (5): 572-77.

Li, et al. (2014) "AxonQuant: A Microfluidic Chamber Culture-Coupled Algorithm That Allows High-Throughput Quantification of Axonal Damage." Neurosignals 22 (1): 14-29.

Muramatsu and Yamashita (2013), "Primary Culture of Cortical Neurons." Bio-protocol 3(8): e496. DOI: 10.21769/BioProtoc.496.

Meijering, (2012)"Cell segmentation: 50 years down thread [life sciences]," IEEE Signal Processing Magazine, vol. 29, no. 5, pp. 140-145.

Ronneberger et al., "U-net: Convolutional networks for biomedical image segmentation," in International Conference on Medical image computing and computer-assisted intervention. Springer, 2015, pp. 234-241.

Simonyan, K., & Zisserman, A. (2014). Very deep convolutional networks for large-scale image recognition. arXiv preprint ar Xiv: 1409.1556.

Whitesides et al., Soft lithography in biology and biochemistry. Annu Rev Biomed Eng. 2001; 3:335-373.

Xiong et al. (2014) J. Adhesion Science and Technology, 28, 1046-1054

Zille et al., "Neuronal Death After Hemorrhagic Stroke In Vitro and In Vivo Shares Features of Ferroptosis and Necroptosis," in Stroke. 2017 Apr;48(4): 1033-1043. doi: 10.1161/STROKEAHA.116.015609. Epub 2017 Mar 1.

**Patentansprüche**

1.  Mikrofluidische Testvorrichtung zur Kultivierung und/oder Untersuchung von Neuronen, umfassend mindestens eine Trägerplatte und einen Körper, wobei der Körper eine Mehrzahl an mikrofluidische Einheiten aufweist, wobei jede mikrofluidische Einheit mindestens zwei sich gegenüberliegende Kammern umfasst, **dadurch gekennzeichnet, dass** der Körper monolithisch ausgestaltet ist.

2.  Mikrofluidische Testvorrichtung nach Anspruch 1, **dadurch gekennzeichnet, dass** die mindestens zwei sich gegenüberliegenden Kammern jeweils ein Kompartiment aufweisen, das sich in Richtung der anderen Kammer erstreckt.

3.  Mikrofluidische Testvorrichtung nach Anspruch 1 oder 2, **dadurch gekennzeichnet, dass** die Kompartimente Mikrokanäle aufweisen.

4.  Mikrofluidische Testvorrichtung nach einem der Ansprüche 2 bis 4, **dadurch gekennzeichnet, dass** die Kompartimente mit der jeweiligen Kammer direkt verbunden sind.

5.  Mikrofluidische Testvorrichtung nach einem der Ansprüche 1 bis 5, **dadurch gekennzeichnet, dass** die Kompartimente über maximal 250 Mikrokanäle miteinander verbunden sind.

6.  Mikrofluidische Testvorrichtung nach einem der Ansprüche 1 bis 6, **dadurch gekennzeichnet, dass** die Kompartimente/und oder die Mikrokanäle eine Überdeckung aufweisen.

7.  Verfahren zur Untersuchung von Neuronen, umfassend die folgenden Schritte:

a) Generieren mikroskopischer Bilddaten von Neuronen in mindestens einer mikrofluidischen Einheit, wobei die Neuronen jeweils in einer mikrofluidischen Einheit vorliegen, wobei von einer mikrofluidischen Einheit mindestens ein mikroskopisches Bild erzeugt wird, wobei jedes mikroskopische Bild eine Mehrzahl an Pixeln aufweist;

b) Generieren von sekundären Bilddaten unter Verwendung eines Bildverarbeitungsalgorithmus, wobei der Bildverarbeitungsalgorithmus für jedes Pixel der in Schritt a) generierten Bilder mindestens eine Klassenzugehörigkeit bestimmt; und

c) Ausgeben und/oder Erhalten automatisch generierter sekundärer Bilddaten zum Zustand der Neurone.

8. Verfahren zur Untersuchung von Neuronen nach Anspruch 7, wobei das Verfahren weiterhin einen der folgenden Schritte umfasst:

cc) Erzeugen von Daten zum Zustand der Neurone aus den sekundären Bilddaten;

d) Zusammenfügen eines digital aufbereiteten Bildes basierend auf den sekundären Bilddaten;

e) Darstellen der automatisch generierten sekundären Bilddaten oder des digital aufbereiteten Bildes auf einer Bildwiedergabevorrichtung; und/oder

f) Ausgeben und/oder Erhalten automatisch generierter tertiärer und/oder quartärer Bilddaten.

9. Verfahren zur Untersuchung von Neuronen nach Anspruch 8, wobei das Generieren der mikroskopischen Bilddaten in Schritt a) das Aufnehmen von mikroskopischen Bilddaten mittels eines Mikroskops umfasst.

10. Verfahren zur Untersuchung von Neuronen nach einem der Ansprüche 7 bis 9, wobei die Bilddaten in Schritt a) und b) mikroskopische Bilddaten von Axonen umfassen.

11. Verfahren zur Untersuchung von Neuronen nach einem der Ansprüche 7 bis 10, wobei der Bildverarbeitungsalgorithmus in Schritt b) einen Bildverarbeitungsalgorithmus umfasst, welcher anhand von mikroskopischen Bilddaten mit bekannten Informationen trainiert wurde.

12. Verfahren zur Untersuchung von Neuronen nach einem der Ansprüche 7 bis 11, wobei das Generieren von sekundären Bilddaten in Schritt b) das Segmentieren der Bilddaten umfasst.

13. Verfahren zur Untersuchung von Neuronen nach einem der Ansprüche 7 bis 12, wobei die sekundären Bilddaten automatisch generierte sekundäre Bilddaten zum Zustand der Neurone umfassen, wobei diese vorzugsweise in Form einer graphischen Darstellung und/oder in Form einer Tabelle vorliegen.

14. System zur automatischen Untersuchung von Neuronen umfassend mindestens eine Testvorrichtung nach einem der Ansprüche 1 bis 6, mindestens ein Mikroskop, und mindestens eine Auswerteeinheit, beispielweise einen Computer, eine Computing Cloud, ein Netzwerk, einen Tabletcomputer und/oder ein Smartphone, wobei die Auswerteeinheit vorzugsweise geeignet ist das Verfahren nach einem der Ansprüche 7 bis 13 auszuführen.

15. Verfahren zur Kultivierung von Neuronen umfassend:

a) Bereitstellen einer mikrofluidischen Testvorrichtung nach einem der Ansprüche 1 bis 6,

b) Aussäen von Neuronen in mindestens eine der Kammern und/oder Kompartimente, so dass mindestens eine angesäte Kammer entsteht,

c) Inkontaktbringen zumindest eines Teils der angesäten Kammern mit einem Kulturmedium,

d) Kultivieren der Neuronen.

Fig. 1

a

b

c

Fig. 2

Fig. 3

Fig. 4

a

Fig. 5

i)

ii)

iii)

iv)

Europäisches Patentamt

European Patent Office

Office européen des brevets

**EUROPÄISCHER RECHERCHENBERICHT**

Nummer der Anmeldung

EP 20 15 2016

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | CECILIA A BRUNELLO ET AL: "Microtechnologies to fuel neurobiological research with nanometer precision", JOURNAL OF NANOBIOTECHNOLOGY, BIOMED CENTRAL, GB, Bd. 11, Nr. 1, 10. April 2013 (2013-04-10), Seite 11, XP021144228, ISSN: 1477-3155, DOI: 10.1186/1477-3155-11-11 * das ganze Dokument * ----- | 1-15 | INV. C12M3/06 C12M1/00 C12M1/34 |
| X | EP 3 494 877 A1 (NMI NATURWISSENSCHAFTLICHES UND MEDIZINISCHES INSTITUT AN DER UNIV TUE) 12. Juni 2019 (2019-06-12) | 1-6,14, 15 | |
| Y | * Absätze [0032], [0034], [0060], [0061], [0074], [0075], [0079] * * Abbildungen 1,5,6,13 * ----- | 1-15 | |
| X | US 2008/257735 A1 (JEON NOO LI [US] ET AL) 23. Oktober 2008 (2008-10-23) | 1-6,14, 15 | |
| Y | * Abbildungen 1-3 * * Absätze [0011], [0012], [0070] * ----- | 1-15 | RECHERCHIERTE SACHGEBIETE (IPC) C12M |
| X | US 2019/151367 A1 (HOWE CHARLES L [US] ET AL) 23. Mai 2019 (2019-05-23) | 1-6,14, 15 | |
| Y | * Abbildungen 1A,3C * * Absatz [0005] * ----- | 1-15 | |
| Y | US 2014/141497 A1 (BALAGADDE FREDERICK [US] ET AL) 22. Mai 2014 (2014-05-22) * Absätze [0010] - [0014], [0097], [0098], [0227] * ----- | 1-15 | |
| Y | US 2018/298317 A1 (INGBER DONALD E [US] ET AL) 18. Oktober 2018 (2018-10-18) * Absätze [0010], [0011], [0155], [0235]; Abbildungen 2A,2B,4 * ----- | 1-15 | |

-/--

Der vorliegende Recherchenbericht wurde für alle Patentansprüche erstellt

| Recherchenort | Abschlußdatum der Recherche | Prüfer |
|---|---|---|
| Berlin | 17. Juni 2020 | Böhm, Ingo |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.........................................................
& : Mitglied der gleichen Patentfamilie, übereinstimmendes Dokument

EPO FORM 1503 03.82 (P04C03)

Seite 1 von 2

**Europäisches Patentamt**
**European Patent Office**
**Office européen des brevets**

**EUROPÄISCHER RECHERCHENBERICHT**

Nummer der Anmeldung

EP 20 15 2016

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DATABASE WPI Week 201535 Thomson Scientific, London, GB; AN 2015-247733 XP002799448, & KR 2015 0036919 A (DAEGU GYEONGBUK INST SCI & TECHNOLOGY) 8. April 2015 (2015-04-08) * Zusammenfassung * ----- | 1 | |
| A | DATABASE WPI Week 201738 Thomson Scientific, London, GB; AN 2017-30157D XP002799449, & WO 2017/078190 A1 (DAEGU GYEONGBUK INST SCI & TECHNOLOGY) 11. Mai 2017 (2017-05-11) * Zusammenfassung * ----- | 1 | |
| A | WIERINGA P A ET AL: "Neural growth into a microchannel network: Towards A regenerative neural interface", NEURAL ENGINEERING, 2009. NER '09. 4TH INTERNATIONAL IEEE/EMBS CONFERENCE ON, IEEE, PISCATAWAY, NJ, USA, 29. April 2009 (2009-04-29), Seiten 51-55, XP031478272, ISBN: 978-1-4244-2072-8 * das ganze Dokument * ----- | 1 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Berlin | 17. Juni 2020 | Böhm, Ingo |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 20 15 2016

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-06-2020

| Im Recherchenbericht angeführtes Patentdokument | Datum der Veröffentlichung | Mitglied(er) der Patentfamilie | Datum der Veröffentlichung |
|---|---|---|---|
| EP 3494877 A1 | 12-06-2019 | EP 3494877 A1 | 12-06-2019 |
| | | WO 2019115320 A1 | 20-06-2019 |
| US 2008257735 A1 | 23-10-2008 | US 2008257735 A1 | 23-10-2008 |
| | | WO 2006037033 A2 | 06-04-2006 |
| US 2019151367 A1 | 23-05-2019 | EP 3439648 A1 | 13-02-2019 |
| | | US 2019151367 A1 | 23-05-2019 |
| | | WO 2017176619 A1 | 12-10-2017 |
| US 2014141497 A1 | 22-05-2014 | EP 1743019 A2 | 17-01-2007 |
| | | JP 2007517523 A | 05-07-2007 |
| | | US 2005164376 A1 | 28-07-2005 |
| | | US 2009018195 A1 | 15-01-2009 |
| | | US 2012065277 A1 | 15-03-2012 |
| | | US 2014141497 A1 | 22-05-2014 |
| | | WO 2005069980 A2 | 04-08-2005 |
| US 2018298317 A1 | 18-10-2018 | AU 2016285700 A1 | 30-11-2017 |
| | | CA 2983821 A1 | 05-01-2017 |
| | | EP 3286297 A2 | 28-02-2018 |
| | | GB 2554283 A | 28-03-2018 |
| | | US 2018298317 A1 | 18-10-2018 |
| | | WO 2017003546 A2 | 05-01-2017 |
| KR 20150036919 A | 08-04-2015 | KEINE | |
| WO 2017078190 A1 | 11-05-2017 | KR 20170051072 A | 11-05-2017 |
| | | WO 2017078190 A1 | 11-05-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **WHITESIDES et al.** *Annu Rev Biomed Eng.,* 2001, vol. 3, 335-373
- U-net: Convolutional networks for biomedical image segmentation. **O. RONNEBERGER ; P. FISCHER ; T. BROX.** International Conference on Medical image computing and computer-assisted intervention. Springer, 2015, 234-241
- **BAHDANAU et al.** Neural machine translation by jointly learning to align and translate. *3rd International Conference on Learning Representations, ICLR 2015 - Conference Track Proceedings,* 2015
- **BEIROWSKI et al.** The Progressive Nature of Wallerian Degeneration in Wild-Type and Slow Wallerian Degeneration (WldS) Nerves. *BMC Neuroscience,* 2005, vol. 6 (1), 6
- **DENG, J. ; DONG, W. ; SOCHER, R. ; LI, L. J. ; LI, K. ; FEI-FEI, L.** Imagenet: A large-scale hierarchical image database. *2009 IEEE conference on Computer vision and pattern recognition,* Juni 2009, 248-255
- **DILLENCOURT et al.** A general approach to connected-component labeling for arbitrary image representations. *Journal of the ACM,* 1992, vol. 39 (2), 253
- **KERSCHENSTEINER ; MARTIN, MARTIN E SCHWAB ; JEFF W LICHTMAN ; THOMAS MISGELD.** In Vivo Imaging of Axonal Degeneration and Regeneration in the Injured Spinal Cord. *Nature Medicine,* 2005, vol. 11 (5), 572-77

- **LI et al.** AxonQuant: A Microfluidic Chamber Culture-Coupled Algorithm That Allows High-Throughput Quantification of Axonal Damage. *Neurosignals,* 2014, vol. 22 (1), 14-29
- **MURAMATSU ; YAMASHITA.** Primary Culture of Cortical Neurons. *Bio-protocol,* 2013, vol. 3 (8), e496
- **MEIJERING.** Cell segmentation: 50 years down thread [life sciences. *IEEE Signal Processing Magazine,* 2012, vol. 29 (5), 140-145
- U-net: Convolutional networks for biomedical image segmentation. **RONNEBERGER et al.** International Conference on Medical image computing and com-puter-assisted intervention. Springer, 2015, 234-241
- **SIMONYAN, K. ; ZISSERMAN, A.** *Very deep convolutional networks for large-scale image recognition,* 2014, 1409-1556
- **WHITESIDES et al.** Soft lithography in biology and biochemistry. *Annu Rev Biomed Eng.,* 2001, vol. 3, 335-373
- **XIONG et al.** *J. Adhesion Science and Technology,* 2014, vol. 28, 1046-1054
- **ZILLE et al.** Neuronal Death After Hemorrhagic Stroke In Vitro and In Vivo Shares Features of Ferroptosis and Necroptosis. *Stroke,* April 2017, vol. 48 (4), 1033-1043